(19) Europäisches Patentamt / European Patent Office / Office européen des brevets

(11) **EP 4 378 450 A1**

(12) **EUROPEAN PATENT APPLICATION**

(43) Date of publication:
 **05.06.2024 Bulletin 2024/23**

(51) International Patent Classification (IPC):
 **A61K 9/00** (2006.01)  **A61K 9/20** (2006.01)
 **A61K 31/05** (2006.01)  **A61K 31/485** (2006.01)
 **A61K 47/10** (2017.01)  **A61K 47/22** (2006.01)

(21) Application number: **23213590.5**

(52) Cooperative Patent Classification (CPC):
 **A61K 9/006; A61K 9/2018; A61K 31/05;**
 **A61K 31/485; A61K 47/10; A61K 47/22**

(22) Date of filing: **01.12.2023**

(84) Designated Contracting States:
 **AL AT BE BG CH CY CZ DE DK EE ES FI FR GB GR HR HU IE IS IT LI LT LU LV MC ME MK MT NL NO PL PT RO RS SE SI SK SM TR**
 Designated Extension States:
 **BA**
 Designated Validation States:
 **KH MA MD TN**

(30) Priority: **01.12.2022 US 202218073347**

(71) Applicants:
 • **Alvit LCS Pharma Ltd.**
 **7404709 Ness Ziona (IL)**

 • **Sela, Yoram**
 **43263 Ra'ananna (IL)**

(72) Inventors:
 • **SELA, Yoram**
 **43263 Ra'ananna (IL)**
 • **LAMENSDORF, Itschak**
 **Modiin (IL)**
 • **COHEN, Nachshol**
 **4222337 Netanya (IL)**

(74) Representative: **Lecomte & Partners**
 **76-78, rue de Merl**
 **2146 Luxembourg (LU)**

(54) **SUBLINGUAL CANNABINOID COMPOSITIONS**

(57) The present invention provides a composition for a sublingual or a buccal delivery of cannabinoid active ingredients comprising a therapeutically effective dose of at least one cannabinoid, an emulsifying and penetration enhancing solubilizer such as vitamin E TPGS, Menthol as a permeation enhancer at 1-50 mg/dose, and polyethylene oxide as a mucoadhesive polymer at 2-100 mg, whereby the composition is administered sublingually or buccally in the form of tablet or film, and whereby the emulsifying and penetration enhancing solubilizer is present in an amount equal to or larger than 50% of the amount of the cannabinoid. The present invention further provides a composition for a sublingual or a buccal delivery of cannabinoid active ingredients comprising a therapeutically effective dose of at least one cannabinoid in a mucoadhesive dosage form, comprising sublingual tablets or films and having greater bioavailability than an alcohol-based oral spray having 5.2mg/per spray of cannabinoid active ingredients.

Figure 2

**Description**

This application claims priority from U. S. Patent Application No. 18/073,347, filed December 1, 2022, which is a Continuation-in-Part of Application No. 16/613,194, filed November 13, 2019, which is a U.S. National Stage entry of PCT International Application No. PCT/IB2018/053307, filed May 11, 2018, which claims priority from Provisional Application No. 62/505,873, filed May 13, 2017. All of these applications are hereby incorporated by reference in their entirety.

FIELD OF THE INVENTION

The present invention relates to novel compositions comprising cannabis botanical extracts, isolated or pure molecules, and synthetic derivatives in a sublingual dosage form exhibiting improved bioavailability, faster onset and reduced side-effects.

BACKGROUND OF THE INVENTION

Sublingual delivery refers to the pharmacological route of administration by which drugs diffuse into the blood through tissues under the tongue. Pharmaceuticals which have thus far been developed for sublingual administration include: cardiovascular drugs, steroids, barbiturates, enzymes, vitamins and minerals.

When an active ingredient comes in contact with the mucous membrane beneath the tongue, it diffuses through it. Advantageously, because the connective tissue beneath the epithelium contains a profusion of capillaries, the substance then diffuses into these capillaries and enters the venous circulation. In contrast, substances absorbed in the intestines are subject to "first pass metabolism" in the liver before entering the general circulation, this being a major cause for low bioavailability of known GI delivered dosage forms.

Sublingual administration has other advantages over GI administration. Being more direct, sublingual delivery may be faster acting, ensuring that the substance risks degradation only by salivary enzymes before entering the bloodstream. In contrast, swallowed drugs (upper GI delivery) must survive passage through the hostile environment of the gastrointestinal tract, risking degradation by stomach acid, bile, or one of the many enzymes therein, such as monoamine oxidase (MAO). Furthermore, as mentioned briefly above, following absorption through the gastrointestinal tract, drugs must pass through the liver, where they may be extensively altered; this is known as the first pass effect of drug metabolism. Therefore, oral or upper G! delivery is often very inefficient and hence unsuitable for some of the most important drugs widely used by patients. Cannabis is one example.

As a result of the more effective absorption that sublingual delivery can effect, it is in some cases possible to reduce the absolute dosage of the drug when sublingually administered.

Several options of sublingual administration include: regular or fast-disintegrating sublingual tablets, lipid matrix sublingual tablets, thin films and sublingual sprays. Unfortunately many of the known sublingual delivery systems suffer from the disadvantages that delivery performance and bioavailability are affected by the physical properties of the active, like solubility, crystal morphology, particle size, hygroscopicity, compressibility and mainly polarity.

Cannabis is a genus of flowering plants, sometimes divided into additional subspecies like cannabis indica and cannabis ruderalis. These three taxa have long been used for fiber (hemp), seed and seed oils, medicinal purposes, and as a recreational drug. Industrial hemp products are made from cannabis plants selected to produce an abundance of fiber. In addition, specific cannabinoids were isolated from the full extract, mainly THC and CBD.

Cannabis strains have been bred to produce desired levels of THC and/or CBD. In some cases, having minimal levels of THC, the principal psychoactive constituent obtained through the dried flowers of cannabis plants, and in other cases to produce high levels of THC and other psychoactive cannabinoids. Various extracts including hashish and hash oil are also produced from the plant.

Nabiximols® (also referred to hereinbelow by its USAN trade name Sativex®) is a patented cannabinoid oromucosal mouth spray developed by the UK company GW Pharmaceuticals for multiple sclerosis (MS) patients, who can use it to alleviate neuropathic pain, spasticity, overactive bladder, and other symptoms. It is also approved in Canada, France and some other European countries for some of the above-mentioned indications. Nabiximols® is also being developed as a potential treatment to alleviate pain associated with cancer, and it has also been researched in various models of peripheral and central neuropathic pain.

Nabiximols® is distinct in that it contains a mixture of compounds derived from cannabis plants, rather than a single molecular synthetic product. Although it is a pharmaceutical product standardized in composition, formulation and dose, Sativex® is still in essence a tincture of the cannabis plant and its principal active cannabinoid components are the cannabinoids: tetrahydrocannabinol (THC) and cannabidiol (CBD). The product is formulated as an oromucosal spray which is administered by spraying into the mouth. Each spray delivers a near 1:1 ratio of CBD to THC, with a fixed dose of 2.7 mg THC and 2.5 mg CBD.

CHEMISTRY AND PHARMACOKINETICS

Cannabinoid pharmacokinetics encompasses absorption via diverse routes of administration and from different drug formulations, analyte distribution throughout the body, metabolism by the liver and extra-hepatic tissues, and elimination in the feces, urine, sweat, oral fluid, and hair. Pharmacokinetic processes are dynamic, may change over time, and may be affected by the frequency and extent of drug exposure. Cannabinoid pharmacokinetics research is challenging due to low analyte concentrations, rapid and extensive metabolism, and physico-chemical characteristics hindering the separation of drugs of interest from biological matrices and from each other. More than 421 different chemical compounds, including over 60 cannabinoids, have been identified or isolated from cannabis *sativa.*

Understanding cannabinoid plant chemistry has proven far more complex than simply looking at pure THC. Different effects may be experienced due to the presence or absence of additional cannabinoids and other chemicals. Eighteen different classes of chemicals, including nitrogenous compounds, amino acids, hydrocarbons, carbohydrates, terpenes, and simple and fatty acids, contribute to the known pharmacological and toxicological properties of cannabis. THC is usually present in cannabis plant material as a mixture of monocarboxylic acids, which readily and efficiently decarboxylate upon heating. THC decomposes when exposed to air, heat, or light; exposure to acid can oxidize the compound to cannabinol (CBN), a much less-potent cannabinoid. In addition, cannabis plants dried in the sun release variable amounts of THC through decarboxylation. The pyrolysis caused by smoking whole or agriculturally sourced cannabis may produce more than 2,000 compounds. Due to the chemical complexity of cannabis plant material compared to synthetic THC, extracts of cannabis that capture the full range of cannabinoids are being explored as therapeutic medications.

While cannabis has been used as medicine for thousands of years, cultivation methods have been developed in recent decades to reproducibly yield plants with defined THC or CBD profiles - i.e. concentrations and ratios.

Two standardized extract preparations are known: Tetranabinex M® which is high in THC, and Nabidiolex M®, which is high in CBD are known. Sativex M® contains nearly equal (i.e. 1:1) proportions of Tetranabinex M® and Nabidiolex M®, and hence, almost equal amounts of THC and CBD. THC and CBD comprise approximately 70% of the active ingredient of the product, with 5% comprising other cannabinoids, and the remainder comprising terpenoids, flavonoids, sterols, alkanes, and other chemicals.

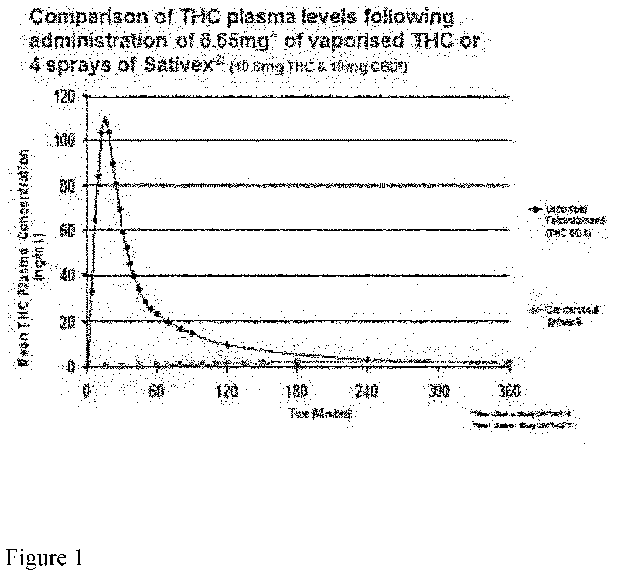

As can be easily observed from **figure 1**, four sprays of the approved oral spray dosage form Sativex® shows very limited bioavailability when compared to vaporised THC. In addition since this spray is an alcoholic spray, patients with sensitive oral mucosa suffer from undesirable irritation and other topical side effects, a severe compliance issue, on top of the product's low bioavailability.

Since oral bioavailability of cannabinoids may be limited, to overcome these obstacles, cannabinoids can be incorporated into oil-in-water nano emulsions: a process known to enhance the delivery of lipophilic bio-actives by making them behave like water-soluble (hydrophilic) compounds.

Emulsion can be categorized into 2 types, oil-in-water emulsion (o/w) and water-in-oil emulsion (w/o). Emulsion is stabilized by adding emulsifying agent. The HLB method (hydrophilic-lipophilic balance) is widely used to determine the quantity and type of surfactant that is needed to prepare a stable emulsion.

Every surfactant is given a number in the HLB scale, that is, from 1 (most lipophilic) to 20 (most hydrophilic), as described in BARRET Roland et al., "Hydrophilic-Lipophilic Balance", From: Handbook for Cleaning/Decontamination of Surfaces, 2007. The calculation formula of HLB data is as follows: HLB value = (quantity surfactant 1)(HLB surfactant 1) + (quantity surfactant 2)(HLB surfactant 2). Usually, a combination of 2 emulsifying agents is used to form a more stable emulsion. The HLB of THC is 0.754.

There is therefore an unmet need for an improved, reliable and reproducible sublingual delivery system, especially when dealing with poorly soluble drugs like cannabinoids.

SUMMARY OF THE INVENTION

The exemplary embodiments of the inventive formulations and methods of treatment made possible by them, provide novel sublingual compositions such as sublingual tablets and films comprising cannabinoid active ingredients, a generic term used herein to widely refer to whole or partial cannabis botanical extracts, purified/isolated cannabinoids and synthetic derivatives of cannabinoids, whole or partial, which contains the entire range of extracted cannabinoids, including specific cannabinoids like THC, CBD and others, formulated into unique sublingual compositions comprising the cannabis botanical extract, Vitamin E TPGS (apparently functioning as an antioxidant in addition to its usual role as emulsifier and enhancer), menthol (or similar permeation enhancer) and mucoadhesive polymers including carbomer, CMC, polyethylene oxides, HPMC, HMC and polyvinylpyrollidone.

The novel compositions provide effective and stable sublingual dosage forms having mucoadhesive properties, enabling superior bioavailability of the active ingredients, thereby reducing side-effects and permitting lower doses than has hitherto been thought to be theraspeutically effective without compromising therapeutic efficacy.

The current invention provides a less or non-irritating sublingual tablet or film with mucoadhesive capabilities and improved bioavailability by including polymers like Carbomer and PVP, which result in prolonging sublingual contact and adherence, as well as permeation enhancers such as Vitamin E TPGS and menthol. In addition, the novel compositions of this invention exhibit improved stability of the cannabinoid active ingredients, due to their being in a dry form as well as, it is believed, possibly due to the presence of Vitamin E TPGS, which has antioxidant properties.

The novel sublingual compositions enable the reduction of the cannabinoid active ingredient dose, and mainly the psychoactive THC active ingredient, by 50% or more, without compromising its therapeutic efficacy as compared to Sativex®.

According to one embodiment, there is provided a method of treatment of neuropathic pain or inflammation by administration to a patient in need thereof of a therapeutically effective amount of a composition of this invention.

Said neuropathic pain or inflammation may result from chemotherapy.

In one embodiment, there is provided a method of treatment of epilepsy by administration to a patient in need thereof of a therapeutically effective amount of a composition of this invention.

According to another embodiment, there is provided a method of treatment of Parkinson disease, seizures, epilepsy, PTSD and the like by administration to a patient in need thereof of a therapeutically effective amount of a composition of this invention.

In another embodiment, there is provided a method of treatment of MS related spasm by administration to a patient in need thereof of a therapeutically effective amount of a composition of this invention.

According to one embodiment, there is provided a method of treatment of cancerrelated pain or inflammation by administration to a patient in need thereof of a therapeutically effective amount of a composition of this invention.

In another embodiment, there is provided a method of treatment of a medical condition by administration to a patient in need thereof of a therapeutically effective amount of the composition of this invention.

According to one embodiment, there are provided novel sublingual adhesive compositions in the form of sublingual tablets or films, exhibiting improved bioavailability and stability, reduced side-effects such as irritation, and optionally lower dosage for the same therapeutic effect, in comparison with the commercially available product Sativex®.

It is an object of this invention to disclose a composition for sublingual or buccal delivery of cannabinoid active ingredients comprising at least one cannabinoid, an emulsifying and penetration enhancing solubilizer, a permeation enhancer, and a mucoadhesive polymer; wherein the composition is administered sublingually or buccally, in the form of a tablet or a film, wherein the emulsifying and penetration enhancing solubilizer is present in an amount equal to or larger than 50% of the amount of the cannabinoid active ingredients, wherein the permeation enhancer comprises menthol in an amount of 1-50 mg per dose, and wherein the mucoadhesive polymer is present in the amount of 2-100 mg.

It is another object of this invention to disclose the composition as defined above, wherein the cannabinoid active ingredients are characterized by at least one of the following: a. comprising tetrahydrocannabinol (THC) and cannabidiol (CBD) in a ratio ranging from 5:95 respectively, to 95:5 respectively; b. comprising THC in an amount between 0.5 and 50 mg; c. comprising CBD in an amount between 0.5 and 50 mg.

It is another object of this invention to disclose the composition as defined in any of the above, wherein the emulsifying and penetration enhancing solubilizer comprises at least one selected from the group consisting of Vitamin E TPGS, fatty acid derivatives, terpenes, cationic surfactants, anionic surfactants, glycerides and derivatives thereof, cyclodextrin derivatives, polyols, and any combination thereof.

It is another object of this invention to disclose the composition as defined in any of the above, wherein the mucoadhesive polymer is selected from the group consisting of carbomer, polyethylene oxide, polyvinylpyrrolidone, chitosan, Hydroxypropyl Methylcellulose (HPMC), Hydroxypropyl cellulose (HPC), and any combination thereof.

It is another object of this invention to disclose the composition as defined in any of the above, further comprising at least one second active ingredient.

It is another object of this invention to disclose the composition as defined in any of the above, wherein the second active ingredient comprises at least one selected from the group consisting of bupropion, buprenorphine, naloxone, methadone, at least one cannabinoid, at least one cannabinoid derivative, and any combination thereof.

It is another object of this invention to disclose the composition as defined in any of the above, wherein the cannabinoid active ingredient has been extracted by an extraction method comprising either supercritical fluid extraction with carbon dioxide ($CO_2$) or extraction with a non-polar solvent.

It is another object of this invention to disclose the composition as defined in any of the above, wherein the non-polar solvent comprises butane.

It is another object of this invention to disclose the composition as defined in any of the above, wherein the cannabinoid active ingredient is selected from the group consisting of THCA and CBDA.

It is another object of this invention to disclose the composition as defined in any of the above, wherein the composition adheres to oral mucosa and buccal mucosa.

It is another object of this invention to disclose the composition as defined in any of the above, wherein the mucoadhesive polymer comprises polyethylene oxide, carbomer, polyvinylpyrrolidone, chitosan or cellulose-based polymers, in an amount ranging from 2-100 mg.

It is another object of this invention to disclose the composition as defined in any of the above, further comprising an antioxidant selected from the group consisting of BHT, BHA, and any combination thereof.

It is another object of this invention to disclose the composition as defined in any of the above, wherein the menthol is dissolved in oil or is in crystal form.

It is another object of this invention to disclose the composition as defined in any of the above, for use in a method of treatment of opioid addiction or opioid dependency, the method comprising administering to a patient in need thereof of a therapeutically effective amount of the composition and optionally an additional active ingredient.

It is another object of this invention to disclose the composition as defined in any of the above, wherein the additional active ingredient comprises at least one selected from the group consisting of benzodiazepine, buprenorphine, naloxone, methadone, bupropion, at least one addiction substance, and any combination thereof.

It is another object of this invention to disclose the composition as defined in any of the above, wherein the at least one addition substance comprises at least one opioid withdrawal compound.

It is another object of this invention to disclose the composition as defined in any of the above, wherein the at least one opioid withdrawal compound comprises bupropion.

It is another object of this invention to disclose the composition as defined in any of the above, wherein the optionally additional active ingredient works synergistically with the at least one cannabinoid.

It is an object of this invention to disclose a composition for sublingual or buccal delivery of cannabinoid active ingredients, comprising at least one cannabinoid active ingredient in a mucoadhesive dosage form, comprising sublingual tablets or films and having greater bioavailability than an alcohol-based oral spray having 5.2mg/per spray of cannabinoid active ingredients.

It is another object of this invention to disclose the composition as defined above, having a plasma profile with an AUC at least 150% greater than the alcohol-based oral spray.

BRIEF DESCRIPTION OF THE FIGURES

Figure 1 is a published chart showing comparative bioavailability of active ingredients delivered by the known oral spray dosage form Sativex® versus vaporized THC.

Figure 2 is a chart showing comparative plasma levels in ng/ml of combined THC and CBD active ingredients delivered by 4 sprays of the known oral spray dosage form Sativex® versus 2 of the inventive tablet dosage forms prepared according to an exemplary embodiment of the present inventive techniques.

Figure 3 is a table taken from the literature showing a listing of compounds which may be considered as representative cannabinoid active ingredients.

Figures 4a-i depict a chromatography assay determining the final concentration of THC in the emulsion. Figure 4a depicts a calibration chromatogram; figures 4b-e depict chromatograms of emulsions disclosed in US 2006/0257463; and figures 4f-i depict chromatograms of emulsions disclosed in the present invention.

Figures 5a-h depict optical microscope photos of the emulsions taken after homogenization. Figures 5a-d depict optic microscope photos of homogenized emulsions disclosed in US 2006/0257463; and figures 5e-h depict optic microscope photos of homogenized emulsions disclosed in the present invention.

Figures 6a-b depict test tubes filled with emulsions taken after homogenization. Figure 6a depicts homogenized emulsions disclosed in US 2006/0257463; and figure 6b depicts homogenized emulsions disclosed in the present invention.

Figures 7a-h depict Malvern Zetasizer Nano Series particle distribution reports of the emulsions. Figures 7a-d depict Malvern Zetasizer Nano Series particle distribution reports of the emulsions disclosed in US 2006/0257463; and figures 7e-h depict Malvern Zetasizer Nano Series particle distribution reports of the emulsions disclosed in the present invention.

DETAILED DESCRIPTION OF EXEMPLARY EMBODIMENTS

The wording herein below is implied in the common meaning of the definitions and statements as known to the versed in the art of pharmaceuticals and polymer science. However, there are several terms that should be understood within the context of inventive techniques, formulations, compositions and treatments as follows:

As used in the specification and claims, the forms "a", "an" and "the" include singular as well as plural references unless the context clearly dictates otherwise.

Further, as used herein, the term "comprising" is intended to mean that the system includes the recited elements, but not excluding others which may be optional in the design of the system, such as fillers and the like. The term "consisting essentially of" is used to define a system that includes the recited elements but exclude other elements that may have an essential significance effect on the performance of the system. "consisting of" shall thus mean excluding more than traces of other elements. Embodiments defined by each of these transition terms are within the scope of this invention.

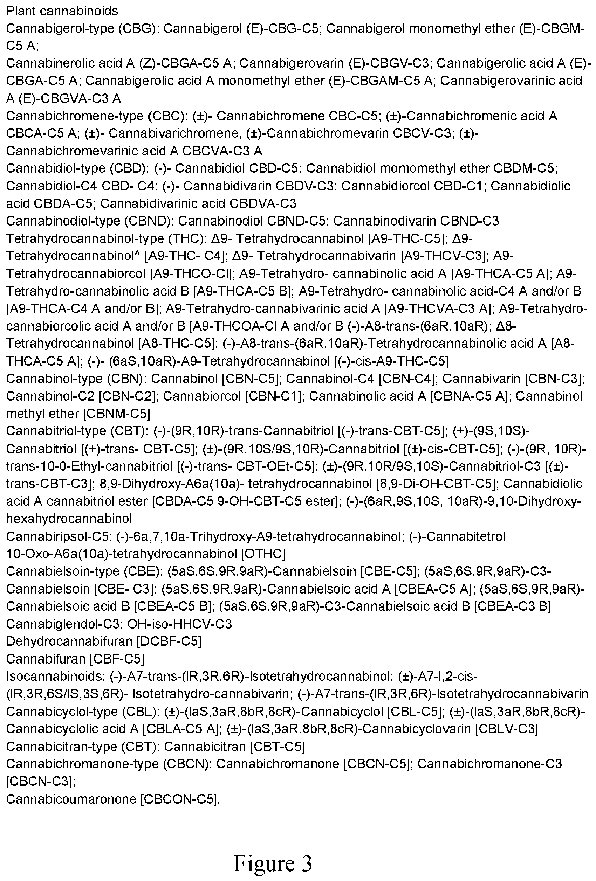

The terms "active", "active pharmaceutical ingredient" and API relate to the pharmaceutical active materials and are used interchangeably. **Figure 3** includes a non-exhaustive list of active ingredients which will be referred to collectively herein as included within the wider and more generic term "cannabinoid active ingredients". The term cannabinoid active ingredients should also be understood as including nonplant derived cannabinoids, cannabinoid-like molecules derived from plants and sources other than Cannabis species, and synthetic derivatives of cannabinoids.

The terms "botanical extracts" and "extracts" relate to a mixture of substances obtained from the plants by an extraction process followed by concentration, and are used interchangeably.

Following are short descriptions of exemplary excipients which may be used in the novel compositions and methods of treatment made possible by the disclosed inventive techniques.

Vitamin E TPGS NF, sourced from Eastman Co., d-$\alpha$-tocopheryl polyethylene glycol 1000 succinate, is a surfactant that is used as an emulsifier, drug solubilizer, absorption enhancer, and as a vehicle for lipid-based drug-delivery formulations. Vitamin E TPGS has found wide utility in pharmaceutical formulations including the following: improvement of drug bioavailability, enhancing solubilization of poorly water-soluble drugs due to its surfactant properties, stabilization of amorphous drug forms and enhancing drug permeability by P-glycoprotein efflux inhibition.

D-$\alpha$-Tocopheryl polyethylene glycol 1000 succinate (simply TPGS or Vitamin E TPGS) is formed by the esterification of Vitamin E succinate with polyethylene glycol 1000. As a nonionic surfactant, it exhibits amphipathic properties and can form micelles in aqueous vehicles. The HLB of Vitamin E TPGS is 13.2. The use of Vitamin E TPGS as surfactant/solubilizer is disclosed for example in US 2006/0257463 [ELSOHLY, Mahmoud] November 11, 2013.

Menthol is an organic compound made synthetically or obtained from cornmint, peppermint or other mint oils. A waxy, crystalline substance, clear or white in color, it is solid at room temperature and melts at temperatures slightly above. The main form of menthol occurring in nature is (-)-menthol, which is assigned the (1R,2S,5R) configuration. Menthol has local anesthetic and counterirritant qualities, and it is widely used to relieve minor throat irritation. Menthol may also act as a weak kappa opioid receptor agonist].

Menthol is included in many pharmaceutical products for a variety of reasons. Its pharmaceutical and medicinal uses are extensive. More relevant for the current application, menthol is useful in transdermal and transmucosal preparations as a permeation enhancer.

Mucoadhesive Polymers

The mucoadhesive polymers used in the novel compositions of this invention exhibit mucoadhesion ability and are selected from the group comprising polyethylene oxide (PEO), carbomer, polyvinylpyrrolidone (povidone, PVP), cellulose based polymers and chitosan in an amount ranging from 2-100 mg per dosage form. HEC, NaCMC, HMC are additional examples of carboxy gels that may be useful for practicing the inventive techniques and preparing the inventive mucoadhesive sublingual dosage forms. See for example Adamo F., et al., Mucoadhesive Gels Designed for the Controlled Release of Chlorhexidine in the Oral Cavity, Pharmaceutics 3, 665-679; 2011 doi:10.3390/pharmaceutics3040665 ISSN 1999-4923, Received: 21 July 2011; in revised form: 9 September 2011 / Accepted: 26 September 2011 / Published: 27 September 2011.This reference describes the use of carboxymethyl- (CMC), hydroxypropylmethyl- (HPMC) and hydroxypropyl- (HPC) cellulose, alone (3% w/w) or in binary mixtures (5% w/w) using chlorhexidine as the active ingredient.

Carbomer (carboxy vinyl polymer also known as carbopol) is a very high molecular weight polymer of acrylic acid with cross linkages of allyl sucrose. Due to the high proportion of the carboxy groups present, carbomer solution is known to be acidic. It is also of low viscosity but when neutralized with triethanolamine, it is converted to highly viscous gels. The adhesive properties of carbomer are exploited to develop mucoadhesive gels and drug delivery systems for controlled and localized drug delivery.

Carbopol polymers have been used worldwide for many years to thicken, modify flow characteristics, emulsify, and suspend insoluble ingredients. Recently, interest in their mucoadhesive properties has grown dramatically.

Mucoadhesion (or muco-adhesion) is generally understood to define the ability of a biological or synthetic material to "stick" to a mucous membrane, resulting in bioadhesion of the material to the tissue for a protracted period of time. This concept has received a significant degree of attention, due to potential applications in drug delivery and enhanced drug bioavailability which results from the lengthened period of time in which the mucoadhesive dosage form is in contact with the absorbing tissue versus a standard dosage form. In order for a material to be mucoadhesive, it must interact with mucus, which is a highly hydrated, viscous anionic hydrogel layer protecting the mucosa. The mucin is composed largely of flexible glycoprotein chains, which are crosslinked. Carbomer is very efficient at this task.

Preparation Of Botanical Cannabis Extracts

The two main cannabinoids present in the various cannabis strains are tetrahydrocannabinol (THC) and cannabidiol (CBD).

Certain cannabis taxa and strains contain these two cannabinoids in various percentages and ratios.

The novel compositions of this invention are prepared from several types of cannabis Botanical Extracts, comprising the two cannabinoids THC and CBD in various ratios, according to therapeutical needs.

The above extracts are obtained either by an extraction process using super critical fluid method (CO 2) extraction, or nonpolar extraction with butane from plant strains producing THC and CBD in specific and reproducible ratios.

Thus, while clones of cannabis strains CN1 are producing about equal amounts of CBD and THC, cannabis strain MM9 produces mostly THC, and HB3 produces mostly CBD. The preparation of the cannabis botanical extracts from these strains is described in Examples 1-6 below.

Preparation Of the Novel Sublingual Mucoadhesive Compositions

The novel sublingual adhesive compositions of this invention in the form of tablet or film are prepared from cannabis botanical extracts of various THC/CBD ratios or pure cannabinoids , Vitamin E TPGS, menthol (crystals or oil), a mucoadhesive polymer (carbomer, PVP, and most hydrogels i.e. those with mucoadhesive characteristics, and other pharmaceutically acceptable inactive ingredients.

The novel compositions comprise cannabis botanical extracts, cannabis isolates, such as purified CBD or THC, their derivatives whether obtained by pyrolysis or entirely synthetic cannabinoids, in a therapeutically effective dose, which is likely to be something significantly less than contained in daily doses of Sativex®.

Examples 1-6 below detail the preparation of compositions comprising a total of between 5-20 mg of the two cannabinoids THC and CBD in various ratios.

For comparison, Sativex® contains a near 1:1 ratio of THC to CBD and is administered as a spray. Each spray puff delivers a fixed dose of 2.7 mg THC and 2.5 mg CBD (a total of 5.2 mg cannabinoids/puff). The treatment includes 5-13 daily puffs, that is 26-67.6 mg cannabinoids THC and CBD/day.

Thus, the compositions of the instant invention contain lower doses of THV/CBD per tablet or film than those used in the Sativex® treatment.

Due to the unique properties of the novel compositions, it is expected that dosages of the novel compositions which are lower than those of the commercially available product Sativex® will lead to comparable or better therapeutic effects.

In one novel application of the exemplary embodiments of the present inventive techniques, one or more additional active ingredient, here buprenorphine HCl and/or naloxone) may be added to the compositions. The combination tablets contain 10 mg of THC/CBD 1:1 and 8 mg buprenorphine HCl.

The preparation of the novel sublingual sublingual compositions is described in Examples 1-6 below.

According to one embodiment, there are provided compositions comprising a therapeutically effective dose of a botanical extract of cannabis, pure cannabinoid isolates or synthetic cannabinoid derivatives, Vitamin E TPGS, menthol, a mucoadhesive polymer, other pharmaceutically acceptable ingredients and optionally an additional active, wherein the composition is administered sublingually in the form of a tablet or film and is mucoadhesive.

The cannabis botanical extracts of this invention comprise between 5-95% THC (tetrahydrocannabinol) and between 5-95% CBD (cannabidiol).

Said cannabis botanical extract may be extracted from the proper cannabis plant strain by an extraction method selected from supercritical fluid extraction with CO2 and extraction with a non-polar solvent.

According to one exemplary embodiment, the sublingual composition of this invention comprises a mucoadhesive excipient.

The mucoadhesive polymer used in the compositions exhibits mucoadhesion ability and is selected from the group comprising PEO, carbomer, PVP, cellulose based polymers and chitosan in an amount ranging from 2-100 mg per dosage form.

Vitamin E TPGS used in the compositions plays the combined role of stabilizer, permeation enhancer and solubilizer. Using TPGS results in a self-emulsified dosage form. The same effect can be achieved with minimal experimentation by one of ordinary skill in the art by using other surface-active materials that lack the permeation enhancement activity of the TPGS in combination with a separate though somewhat less versatile permeation enhancer. As shown in the assay disclosed below comparing the formulation of the present invention to the one available in the prior art, the optimal amount of Vitamin E TPGS in the combination should by at least 50% of the amount of cannabinoids, and preferably equal to or greater than the amount of cannabinoids.

The novel composition may further comprise an antioxidant selected from BHT, BHA and their mixtures.

The sublingual mucoadhesive compositions comprise menthol as oil or crystals in an amount of 1-50 mg per dosage form.

According to one embodiment, there is provided a method of treatment of opioid addiction and dependency by administration to a patient in need thereof of a therapeutically effective amount of a composition of this invention, comprising a therapeutically effective dose of a botanical extract of cannabis and optionally an additional active. One example of an optionally added active ingredient may be buprenorphine HCl. Buprenorphine HCl and the two actives CBD and THC work synergistically as an anti-opioid anti-addiction and dependency combination product.

Reference is now made to a composition for sublingual or buccal delivery of cannabinoid active ingredients comprising at least one cannabinoid, an emulsifying and penetration enhancing solubilizer, a permeation enhancer, and a mucoadhesive polymer; characterized by the composition being administered sublingually or buccally, in the form of a tablet or a film, also characterized by the emulsifying and penetration enhancing solubilizer being present in an amount equal to or larger than 50% of the amount of the cannabinoid active ingredients, also characterized by the permeation enhancer comprising menthol in an amount of 1-50 mg per dose, and also further characterized by the mucoadhesive polymer being present in the amount of 2-100 mg.

Further reference is now made to the above-mentioned composition as defined above, further characterized by the cannabinoid active ingredients being at least one of the following: a. comprising tetrahydrocannabinol (THC) and cannabidiol (CBD) in a ratio ranging from 5:95 respectively, to 95:5 respectively; b. comprising THC in an amount between 0.5 and 50 mg; c. comprising CBD in an amount between 0.5 and 50 mg.

Further reference is now made to the above-mentioned composition as defined in any of the above, further characterized by the emulsifying and penetration enhancing solubilizer comprising at least one selected from the group consisting of Vitamin E TPGS, fatty acid derivatives, terpenes, cationic surfactants, anionic surfactants, glycerides and derivatives thereof, cyclodextrin derivatives, polyols, and any combination thereof.

Further reference is now made to the above-mentioned composition as defined in any of the above, further characterized by the mucoadhesive polymer being selected from the group consisting of carbomer, polyethylene oxide, polyvinylpyrrolidone, chitosan, Hydroxypropyl Methylcellulose (HPMC), Hydroxypropyl cellulose (HPC), and any combination thereof.

Further reference is now made to the above-mentioned composition as defined in any of the above, further characterized by the above-mentioned composition comprising at least one second active ingredient.

Further reference is now made to the above-mentioned composition as defined in any of the above, further characterized by the second active ingredient comprising at least one selected from the group consisting of bupropion, buprenorphine, naloxone, methadone, at least one cannabinoid, at least one cannabinoid derivative, and any combination thereof.

Further reference is now made to the above-mentioned composition as defined in any of the above, further characterized by the cannabinoid active ingredient being extracted by an extraction method comprising either supercritical fluid extraction with carbon dioxide ($CO_2$) or extraction with a non-polar solvent.

Further reference is now made to the above-mentioned composition as defined in any of the above, further characterized by the non-polar solvent comprising butane.

Further reference is now made to the above-mentioned composition as defined in any of the above, further characterized by the cannabinoid active ingredient being selected from the group consisting of THCA and CBDA.

Further reference is now made to the above-mentioned composition as defined in any of the above, further characterized by the composition adhering to oral mucosa and buccal mucosa.

Further reference is now made to the above-mentioned composition as defined in any of the above, further characterized by the mucoadhesive polymer comprising polyethylene oxide, carbomer, polyvinylpyrrolidone, chitosan or cellulose-based polymers, in an amount ranging from 2-100 mg.

Further reference is now made to the above-mentioned composition as defined in any of the above, further comprising an antioxidant selected from the group consisting of BHT, BHA, and any combination thereof.

Further reference is now made to the above-mentioned composition as defined in any of the above, further characterized by the menthol being dissolved in oil or being in crystal form.

Further reference is now made to the above-mentioned composition as defined in any of the above, for use in a method of treatment of opioid addiction or opioid dependency, the method comprising administering to a patient in need thereof of a therapeutically effective amount of the composition and optionally an additional active ingredient.

Further reference is now made to the above-mentioned composition as defined in any of the above, further characterized by the additional active ingredient comprising at least one selected from the group consisting of benzodiazepine, buprenorphine, naloxone, methadone, bupropion, at least one addiction substance, and any combination thereof.

Further reference is now made to the above-mentioned composition as defined in any of the above, further characterized by the at least one addition substance comprising at least one opioid withdrawal compound.

Further reference is now made to the above-mentioned composition as defined in any of the above, further characterized by the at least one opioid withdrawal compound comprising bupropion.

Further reference is now made to the above-mentioned composition as defined in any of the above, further characterized by the optionally additional active ingredient working synergistically with the at least one cannabinoid.

Reference is now made to a composition for sublingual or buccal delivery of cannabinoid active ingredients, comprising at least one cannabinoid active ingredient in a mucoadhesive dosage form, comprising sublingual tablets or films and having greater bioavailability than an alcohol-based oral spray having 5.2mg/per spray of cannabinoid active ingredients.

Further reference is now made to the above-mentioned composition as defined above, further characterized by a plasma profile with an AUC at least 150% greater than the alcohol-based oral spray.

PREPARATION OF THE SAMPLES ACCORDING TO US 2006/0257463, and ALVIT PATENT APPLICATION

Emulsion is a two-phase system that is not stable thermodynamically. It contains at least two immiscible liquids where one of them (internal/dispersed phase) is dispersed homogenously in another liquid (external/continuous phase). The oil phases contained the lipophilic emulsifiers. Primary water-in-oil (W/O) or oil-in-water (O/W) emulsions were prepared by mixing the internal water phase.

The main goals of the current project are:

i. The source of cannabinoids is the extract containing THC (69.18%).

ii. Estimate influence of surfactants, oils and other components on emulsification process.

iii. Compare droplet sizes and size distribution for different formulations.

The proposed approach to the development of emulsion is a two-phase system formulation THC and Vitamin E TPGS includes:

1. Preparation of samples concentrate comprising required amounts of active ingredients with surfactants.

2. Formation of emulsion by combining of the concentrate with water phase.

Following oil phase components were investigated:

**Oil components**

- THC - Oil
  HLB - 0.754

***SURFACTANTS***

- TPGS (Tocopherol succinate polyethylene glycol ester)

  HLB - 13.2
  Antares Vitamin E TPGS has amphiphilic properties with each molecule consisting of polar hydrophilic (polyethylene glycol) and non-polar lipophilic (phytyl chain of d-$\alpha$-tocopherol) moieties.

PARTICLE SIZE ANALYSIS

Particle size analysis was carried out using Malvern Zetasizer Nano-S at 38°C. The process of the micro/nano emulsion is controlled by dispersion particle size measurement using Malvern Zetasizer Nano Series. The final product is also characterized by this instrument.

EQUIPMENT

- Hielscher UP200S ultrasonic processor with tips S7 and S40.
- Ultrasound at 35% amplitude was applied for 10 sec in continuous mode on mixture 5 repeat 3 times, with 10 seconds breaks between cycles.
- Optical microscope (Primo Star, Carl Zeiss, Germany) and microscope digital camera (Moticam 2306).

RESULTS AND DISCUSSION

A brief overview of this experiment will focus on making cannabis THC emulsions via sonication. Initially, response surface methodology (RSM) was performed to optimize the formulation variables of oil-in-water (o/w) emulsions induced by ultrasound.

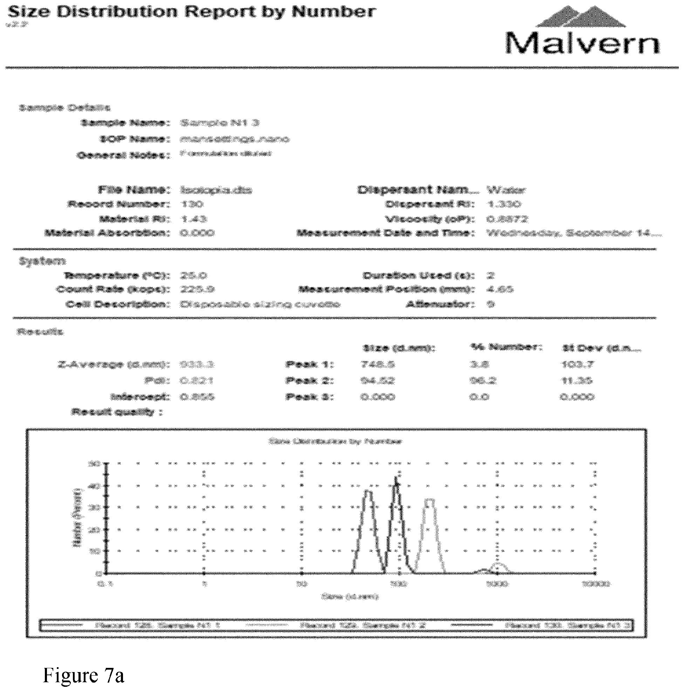

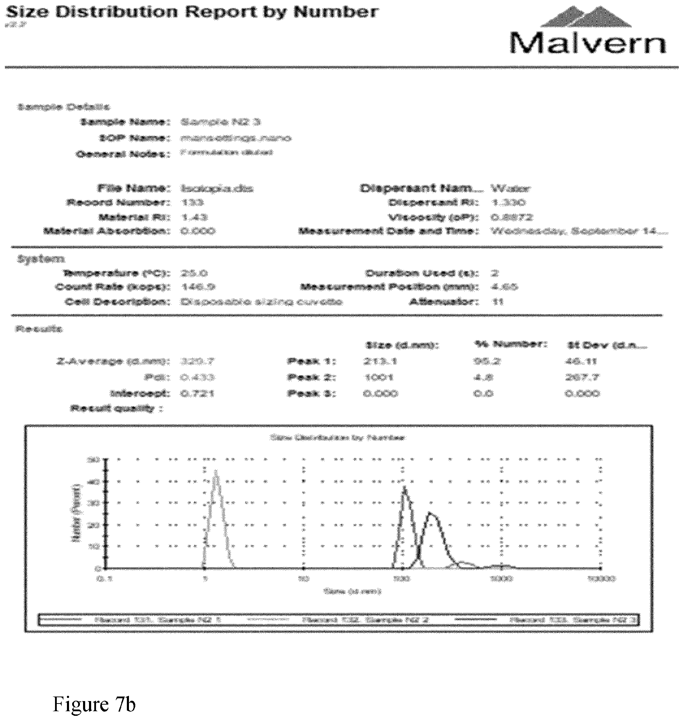

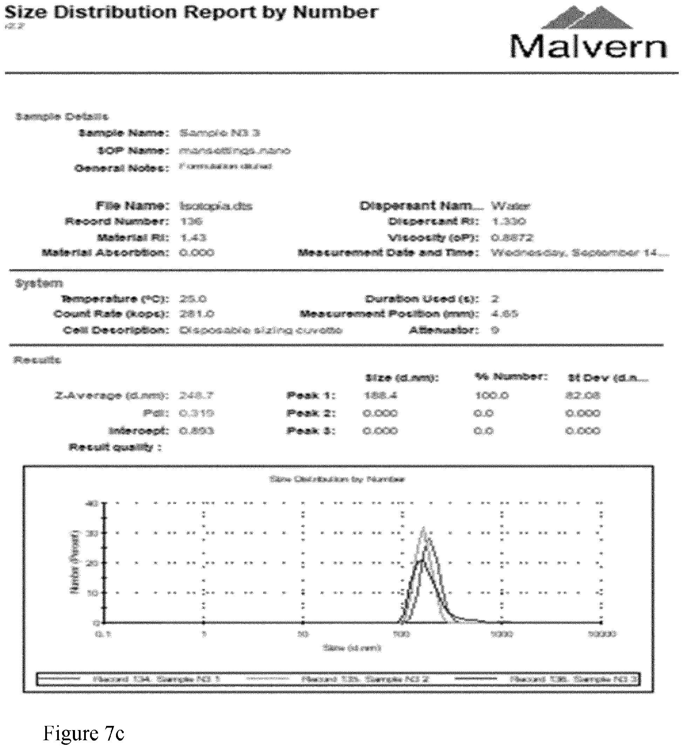

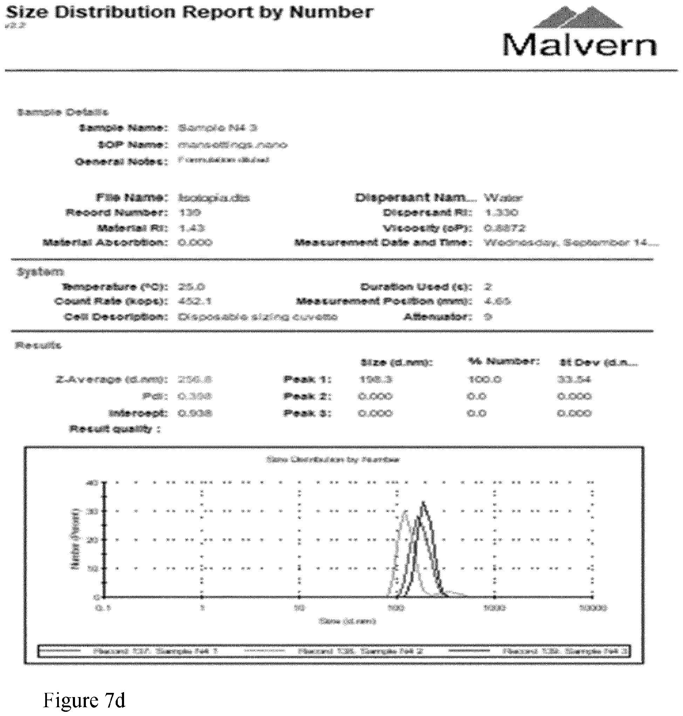

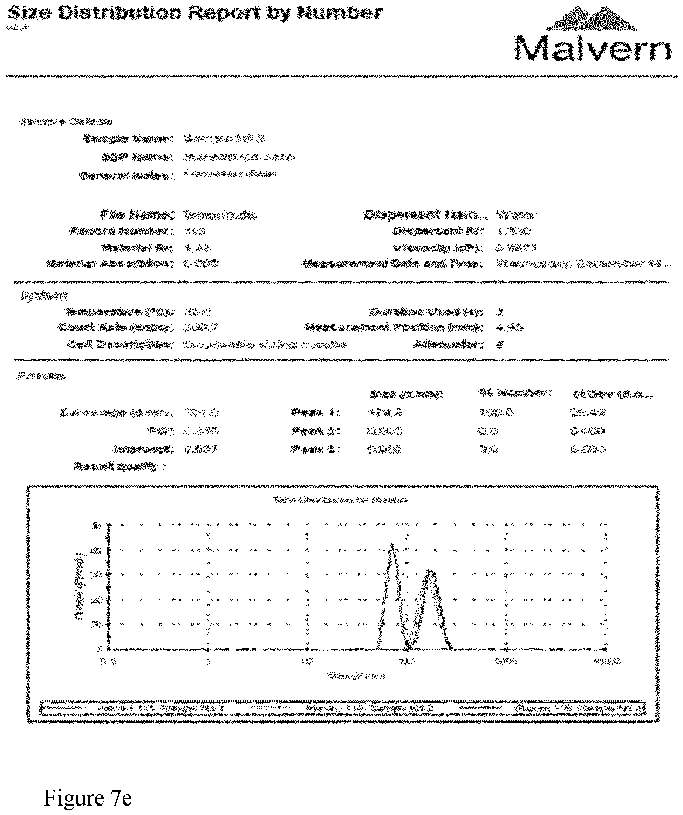

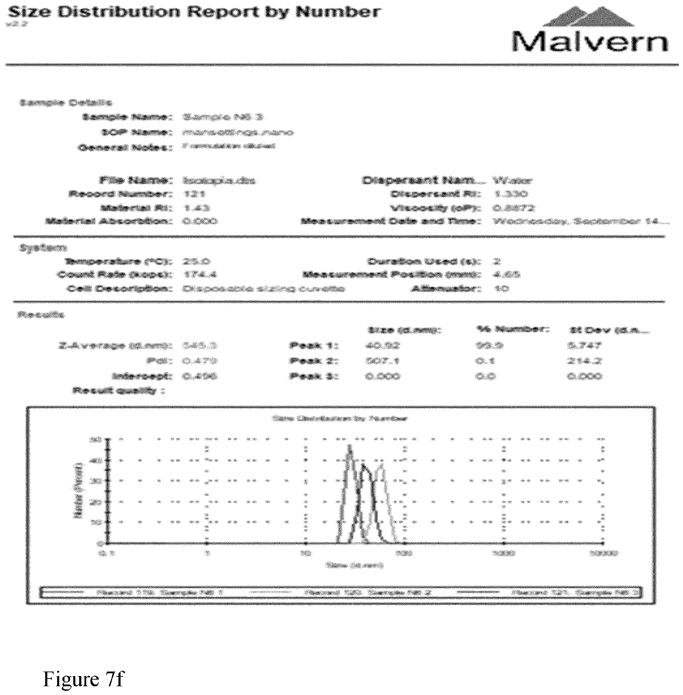

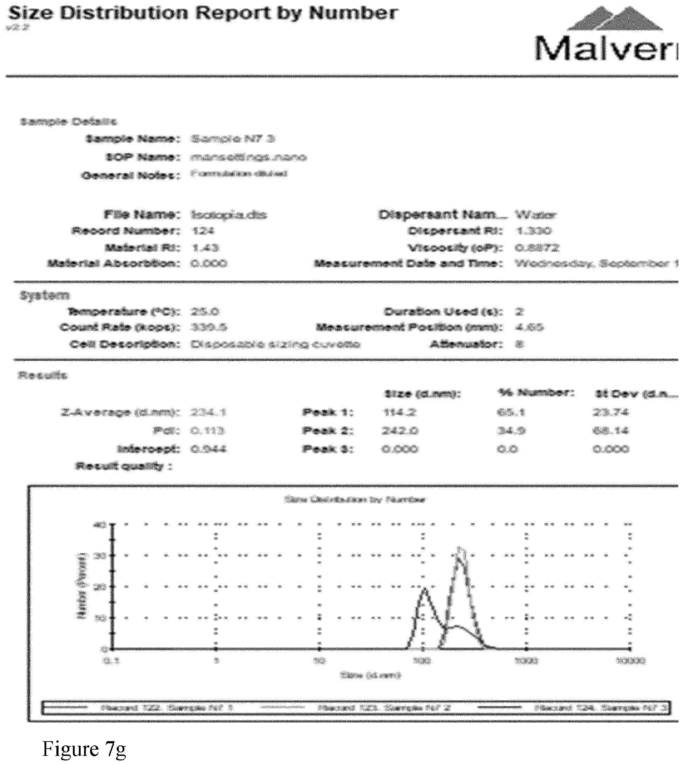

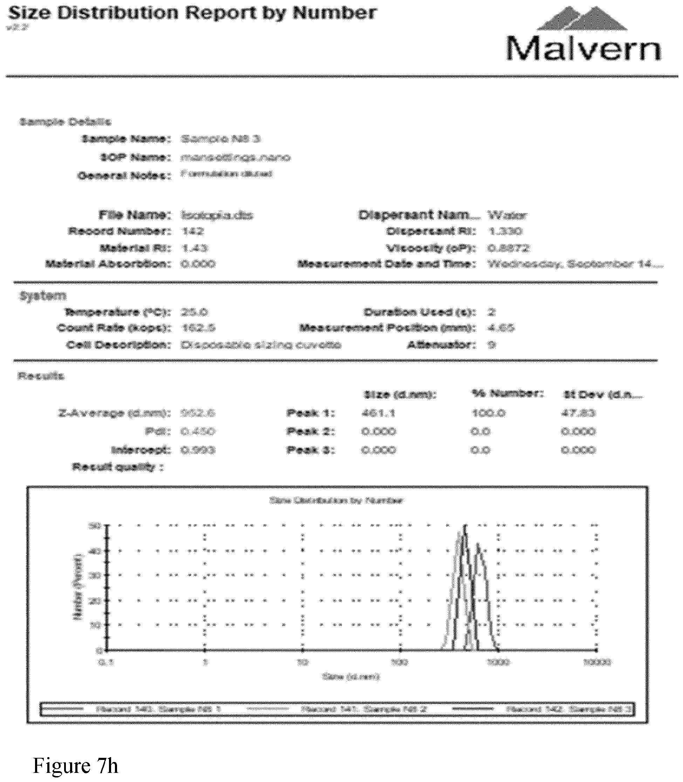

TCH Cannabis oil and Vitamin E TPGS were chosen as the dispersed phase (oil) for oil-in-water (o/w) emulsion. For reducing the interfacial tension between oil and water, the type and amount of emulsifying agent have selected based on previous data and their HLB value. After ultrasonication, the mean particle diameter was measured using dynamic light scattering (DLS) by a Zetasizer Nano (Malvern). See **figures 7a-h.** Deionized water was used for the preparation of all formulations (Thermo Fisher).

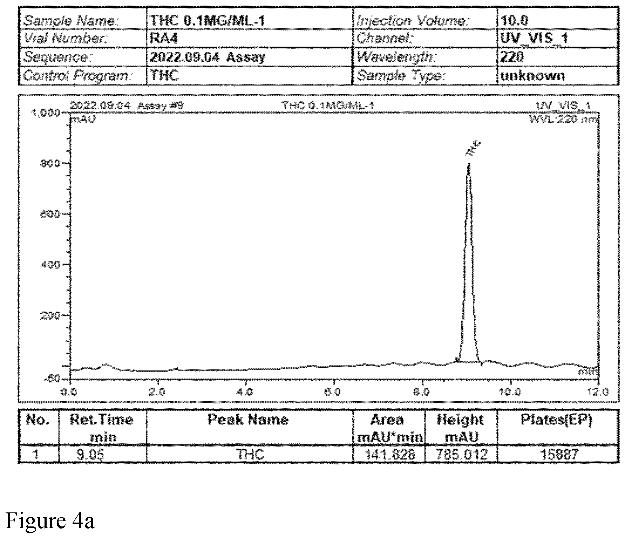

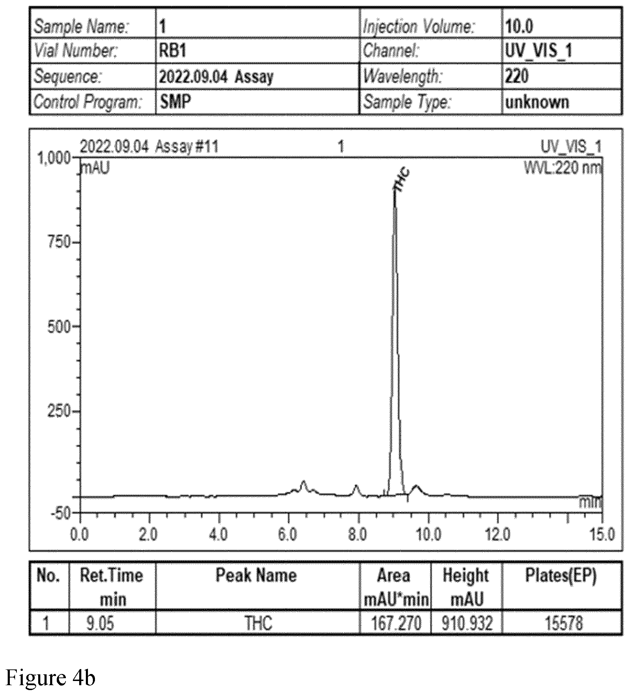

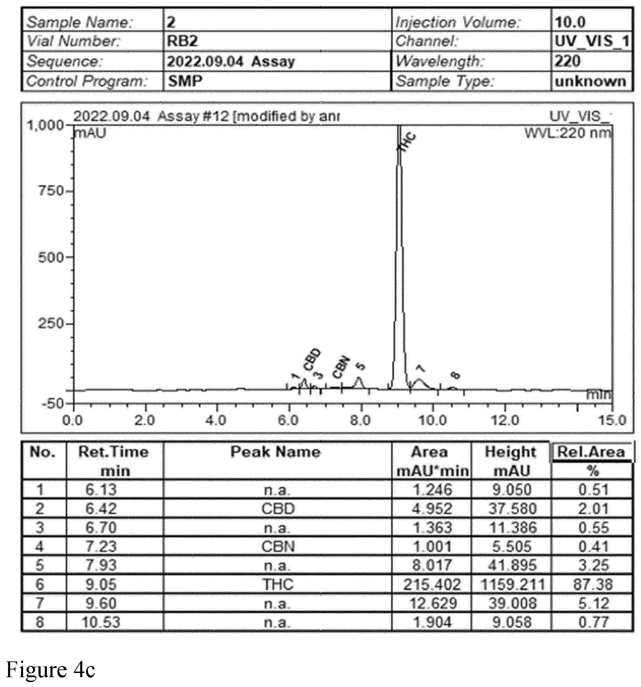

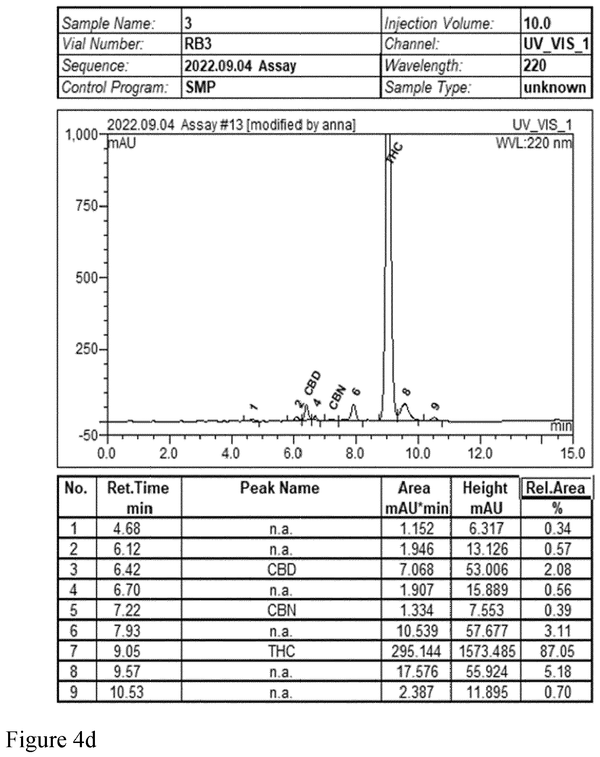

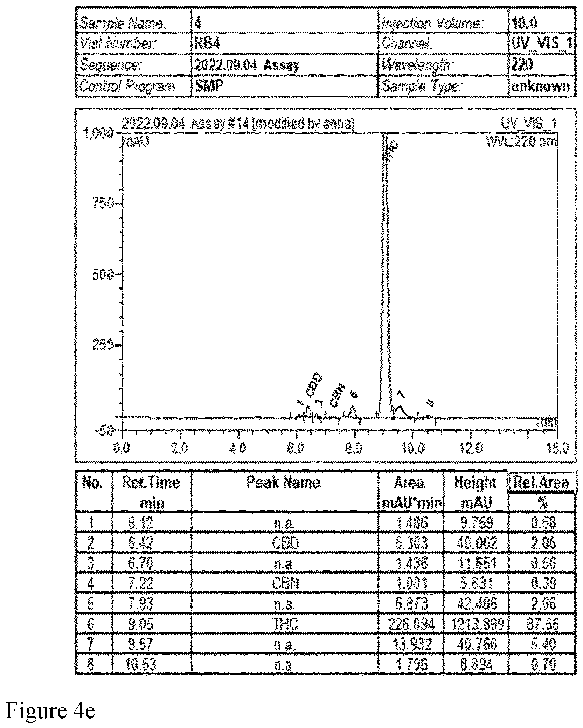

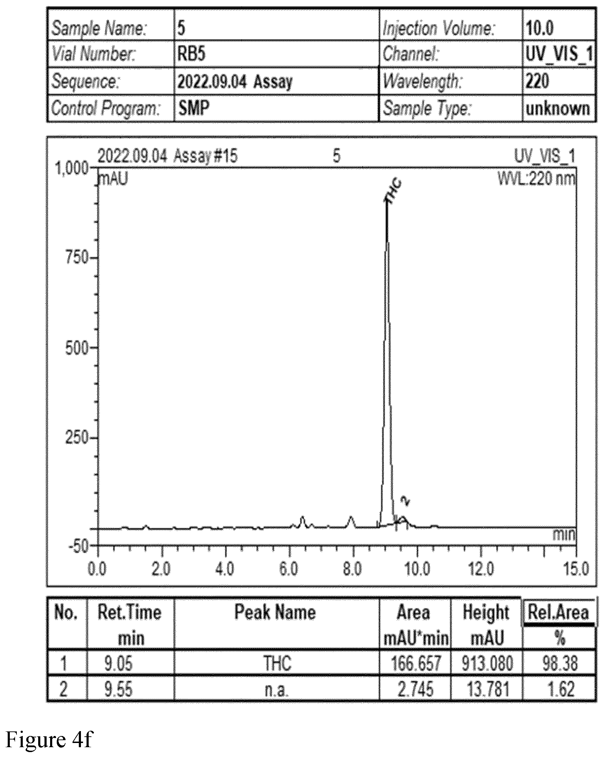

Table 1 below discloses the % of THC in the emulsion of each sample, as derived from a chromatography assay, see **figure 4a-i. Figure 4a** discloses calibration chromatogram of THC. **Figures 4b-e** are chromatograms of samples 1-4, disclosed in US 2006/0257463. **Figures 4f-i** are chromatograms of samples 5-8, disclosed in the present application, see **examples 1-4.**

| Sample Name | THC, % | THC theoretical, % |
|---|---|---|
| 1 | 42.61 | 51 |
| 2 | 62.70 | 68 |
| 3 | 60.06 | 69 |
| 4 | 62.06 | 69 |
| 5 | 40.02 | 35 |
| 6 | 24.86 | 23 |
| 7 | 18.18 | 17 |
| 8 | 41.40 | 47 |

EQIPMENT OF TETRAHYDROCANNABINOL IN OIL COMPOSIOTIONS CHROMOTOGRAPHY ASSAY:

- HPLC analysis was performed on Summit Dionex (Germany) HPLC system with photodiode array (PDA) detectors and Chromeleon Version 6.80 software packages;
- Column: LiChrospher® 60 RP-select B (5$\mu$m) 250x4 mm;
- Mobile phase composition: 80% acetonitrile and 20% Water;
- Flow rate: 1.0 mL/min;
- Column temperature: 30°C;
- Detection wavelength: 220 nm;
- Injection volume: 10 $\mu$L;
- Run time: 15 min.

Emulsions were formulated using oil, surfactants, water and sonication. The dispersed phase (THC oil cannabis/Vitamin E TPGS) was heated up to 45.0 °C and allowed to cool (0.5 hr) in a nitrogen atmosphere for a complete dissolution of surfactant, decarboxylation of cannabinoids and protection from degradation. The continuous phase (water) was also heated up to 45 °C. The emulsification was carried out using a homogenization process.

Ultrasonic exposure was applied in a continuous process. The experiment was repeated with all samples, surfactant fractions, oil fractions, high-power amplitudes and constant hydrophilic lipophilic balance (HLB) values.

Table 2 - Compositions disclosed in US 2006/0257463:

| Number | | Vitamin E TPGS | | THC | |
|---|---|---|---|---|---|
| Example in spec. | Sample | % in formula | % w/w | % in formula | % w/w |
| 5,6 | 1 | 3 | 27.27 | 8 | 72.73 |
| 2 | 2 | 0.1 | 2.44 | 4 | 97.56 |
| 3 | 3 | 0.1 | 1.23 | 8 | 98.77 |
| 4 | 4 | 0.1 | 0.62 | 16 | 99.38 |

HLB values calculation of samples disclosed in prior art:

$$\text{HLB Value (sample 1)} = (27.27 \times 13.2) + (72.73 \times 0.754)/100 = 4.14$$

$$\text{HLB Value (sample 2)} = (2.44 \times 13.2) + (97.56 \times 0.754)/100 = 1.06$$

$$\text{HLB Value (sample 3)} = (1.23 \times 13.2) + (98.77 \times 0.754)/100 = 0.91$$

$$\text{HLB Value (sample 4)} = (0.62 \times 13.2) + (99.38 \times 0.754)/100 = 0.83$$

Table 3 - Compositions disclosed in present invention (see below):

| Number | | Vitamin E TPGS | | THC | |
|---|---|---|---|---|---|
| Example in spec. | Sample | % in formula | % w/w | % in formula | % w/w |
| 1 | 5 | 1 | 50.00 | 1 | 50.00 |
| 2 | 6 | 2 | 66.67 | 1 | 33.33 |
| | | | | | |
| 3 | 7 | 3 | 75.00 | 1 | 25.00 |
| 4 | 8 | 4 | 33.30 | 8 | 67.70 |

HLB values calculation of samples disclosed in present invention:

$$\text{HLB Value (sample 5)} = (33.3 \times 13.2) + (67.7 \times 0.754)/100 = 4.91$$

$$\text{HLB Value (sample 6)} = (50.0 \times 13.2) + (50.0 \times 0.754)/100 = 6.98$$

$$\text{HLB Value (sample 7)} = (66.67 \times 13.2) + (33.3 \times 0.754)/100 = 9.05$$

$$\text{HLB Value (sample 8)} = (75.0 \times 13.2) + (25.0 \times 0.754)/100 = 10.1$$

CONCLUSIONS:

Feasibility investigations show that formulations disclosed in US 2006/0257463 (samples 1-4) contain water in oil (W/O) emulsions.

On the other hand, emulsions disclosed in present invention (samples 5-8) contain oil in water (O/W) emulsion, and demonstrate emulsion behavior upon delusion with water. Oil in water emulsion have better bioavailability, enabling the surfactant Vitamin E TPGS to enhance the delivery of lipophilic bio-actives by making them behave like water-soluble (hydrophilic) compounds.

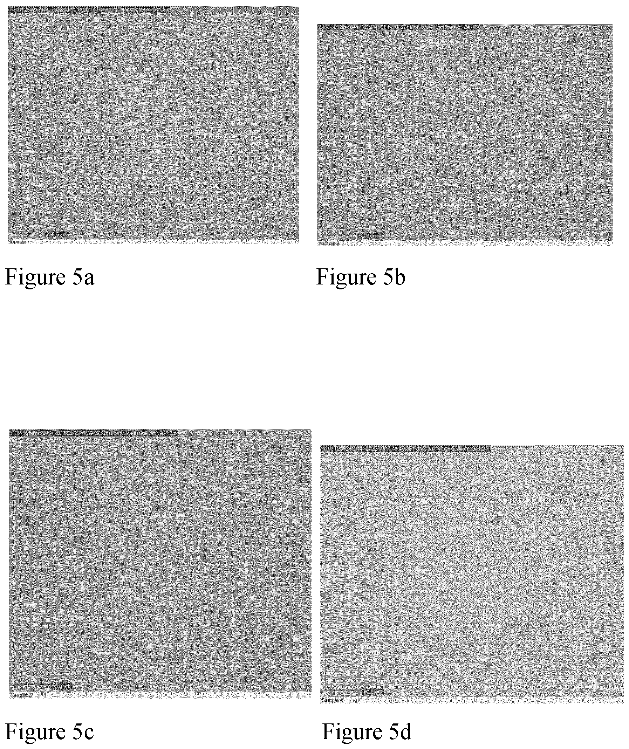

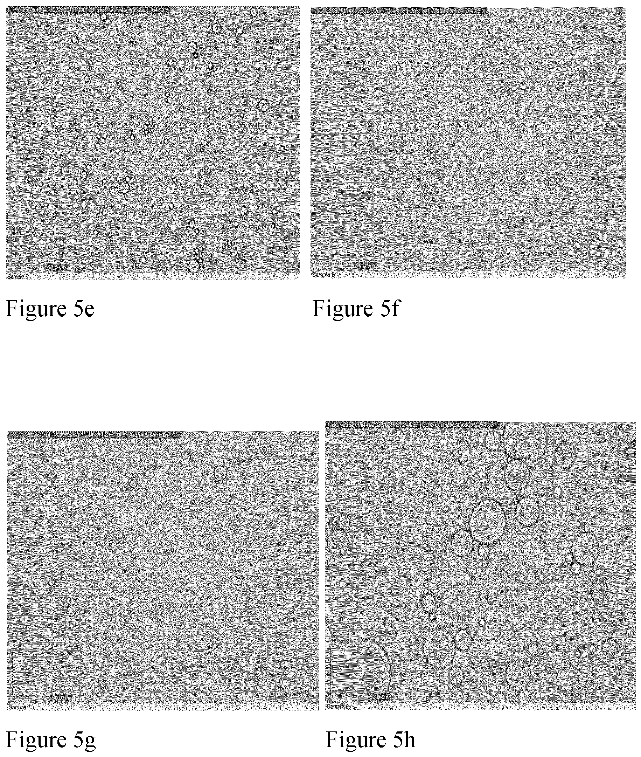

Further reference is being made to **figure 5a-h.** Freshly prepared W/O/W emulsions were analyzed using an optical microscope (Primo Star, Carl Zeiss, Germany) at room temperature. The photo micrograph images of the emulsions were acquired using a microscope digital camera (Moticam 2306) equipped with vision imaging software (Motic Images Plus 2.0). Samples were placed on microscopic slides and then carefully covered with a cover slip to minimize destruction of the emulsion structures. The microstructures of the W/O/W emulsions were observed using an oil immersion objective ($40\times$ magnification), and an appropriate light intensity was selected to reduce sample heating. For each sample, at least three images were taken, and a representative image is shown. Regarding the effects of operation parameters on the formation of W/O/W emulsions, see also WANG Jun, SHI Aimin, AGYEI Domonic, WANG Qiang, "Formulation of water-in-oil-in-water (W/O/W) emulsions containing trans-resveratrol", RSC Adv., 2017, 7, pages 35917-35927.

As can be seen from photo on microscope slides **(figure 5a-h,)** it was found that there were significant differences in the droplet sizes of W/O/W emulsions prepared with different O/W/Solubilizer ratios. This table shows that increasing the O/W/S ratio increases the droplet size significantly. As observed from the microstructures of the emulsions disclosed in US 2006/0257463 (samples 1-4), the oil phase contains smaller water droplets at a W/O/S ratio of example 1(3:8), 2 (0.1:4), 3 (0.1:8), and 4 (0.1:16).

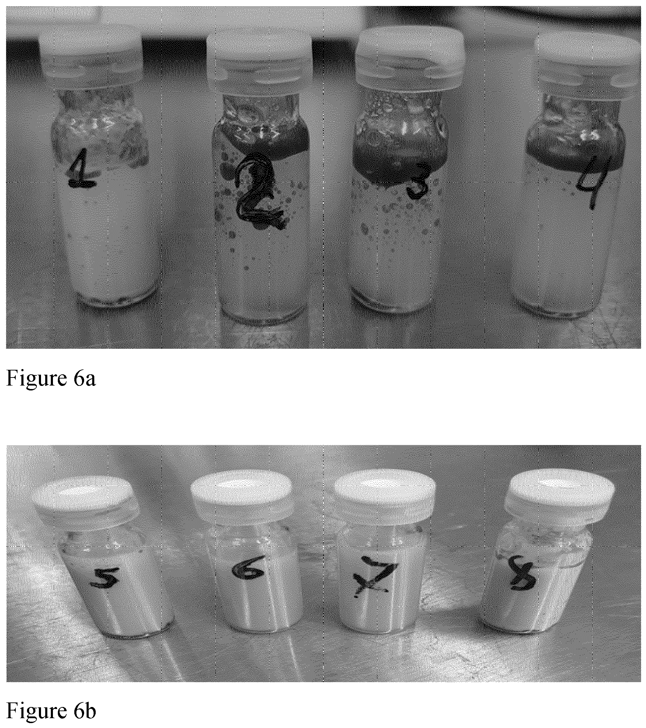

Further reference is being made to **figure 6a-b,** depicting the samples immediately after homogenization. As the internal water phase increases, forming the W/O/W emulsions becomes challenging because there is less emulsifier by proportion, which causes higher surface tension of the droplets. Thus, the W/O emulsions will be unstable. When adding the W/O emulsions to external water phase, it is possible that the internal and external water phases will come together. With homogenization, a large amount of O/W emulsion is formed rather than the desired W/O/W emulsions; this causes a reduction in the droplet sizes of the W/O/W emulsions. See **figure 6a,** depicting the emulsions disclosed in US 2006/0257463 (samples 1-4) after homogenization. It is plain to see that these emulsions do not appear homogenous.

As the internal water phase increases, the oil droplets imbibe larger water droplets, which results in an increase in the droplet sizes of W/O/W emulsions as in the samples 5 (1:1), 6 (2:1), 7 (3:1) and 8 (4:1) disclosed in the present invention **(examples 1-4).** See **figure 6b,** depicting the emulsions the present invention after homogenization, which appear homogenous.

EXEMPLARY EMBODIMENTS

The following examples further illustrate the invention as it may be carried out but, of course, should not be construed as in any way limiting its scope. The scope of the inventive techniques should of course be only understood as limited to and with reference to the claims.

EXAMPLE 1 ("Sample 5" - See **figures 4f, 5e, 6b, 7e)**

1:1 THC/CBD & Vitamin E TPGS

Blend the THC/CBD 1:1 cannabis extract (200 gr of the 70% w/w mixture) together with 800 gr mannitol/lactose 1:1 mixture, 30 gr PVP K-30, 200 gr Vitamin E TPGS, granulate with 600 gr ethanol USP and then dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 10 gr carbomer 934, 20 gr corn starch and 10 gr magnesium stearate. Prepare sublingual THC/CBD 1:1 cannabis extract tablets containing 10 mg of THC/CBD from the above mixture.

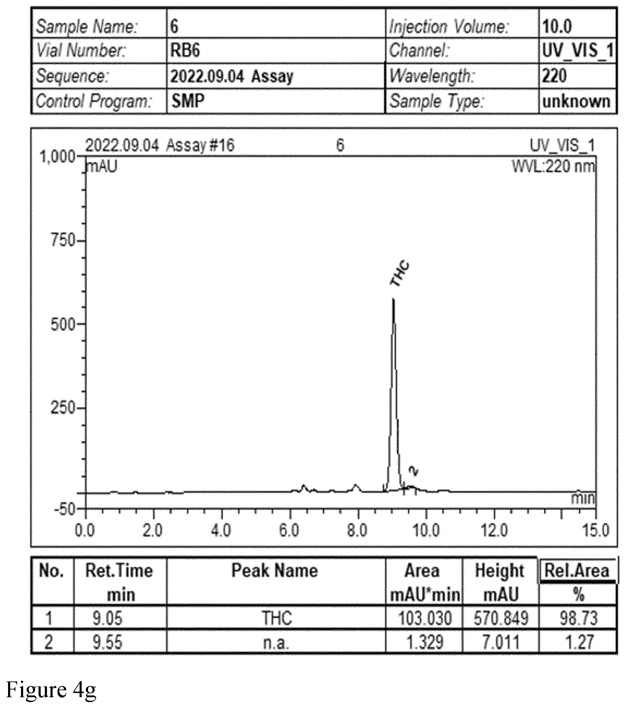

EXAMPLE 2 ("Sample 6" - See **figures 4g, 5f, 6b, 7f)**

2:1 THC/CBD & Vitamin E TPGS

Blend the THC/CBD 2:1 cannabis extract (133.32 gr of the 70% w/w mixture) together with 800 gr mannitol/lactose 1:1 mixture, 30 gr PVP K-30, 266.7 gr Vitamin E TPGS, granulate with 600 gr ethanol USP and then dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 10 gr carbomer 934, 20 gr corn starch and 10 gr magnesium stearate. Prepare sublingual THC/CBD 1:1 cannabis extract tablets containing 10 mg of THC/CBD from the above mixture.

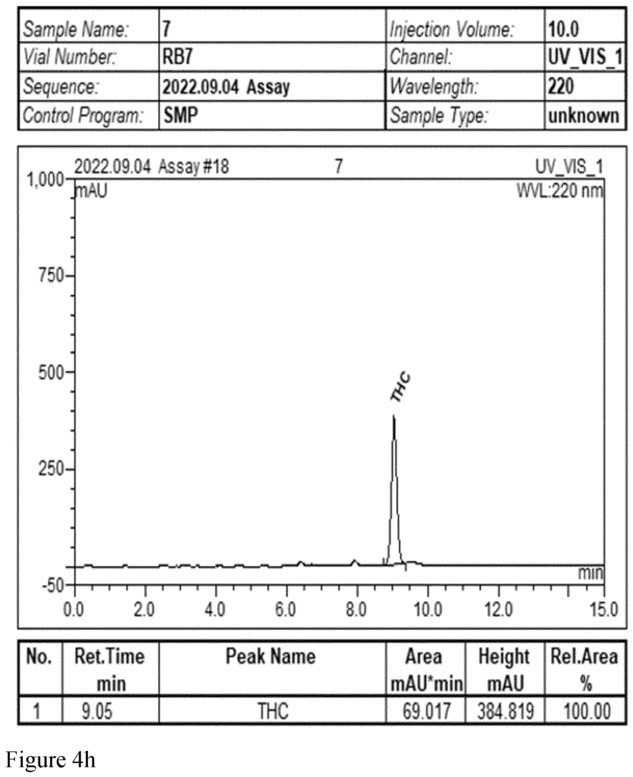

Example 3 ("Sample 7" - See **figures 4h, 5g, 6b, 7g**)

3:1 THC/CBD & Vitamin E TPGS

Blend the THC/CBD 3:1 cannabis extract (100 gr of the 70% w/w mixture) together with 800 gr mannitol/lactose 1:1 mixture, 30 gr PVP K-30, 300 gr Vitamin E TPGS, granulate with 600 gr ethanol USP and then dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 10 gr carbomer 934, 20 gr corn starch and 10 gr magnesium stearate. Prepare sublingual THC/CBD 1:1 cannabis extract tablets containing 10 mg of THC/CBD from the above mixture.

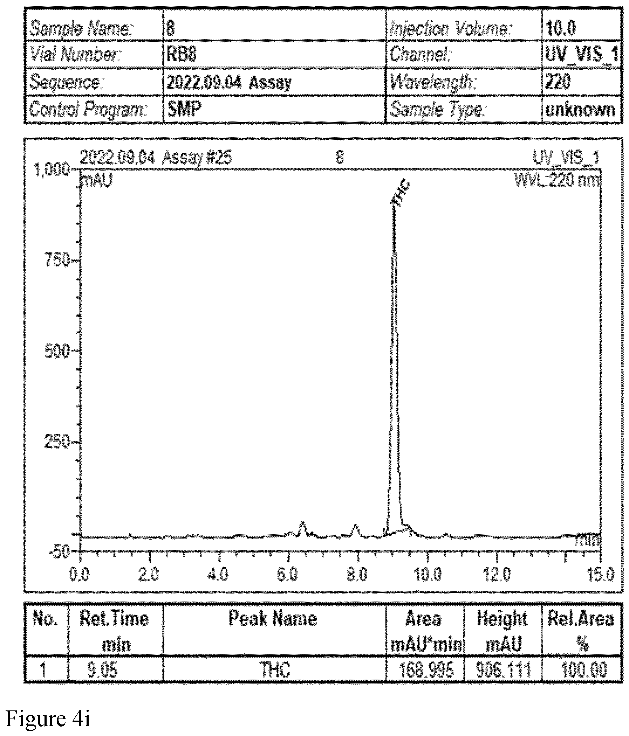

Example 4 ("Sample 8" - See **figures 4i, 5h, 6b, 7h**)

4:1 THC/CBD & Vitamin E TPGS

Blend the THC/CBD 4:1 cannabis extract (266.8 gr of the 70% w/w mixture) together with 800 gr mannitol/lactose 1:1 mixture, 30 gr PVP K-30, 133.2 gr Vitamin E TPGS, granulate with 600 gr ethanol USP and then dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 10 gr carbomer 934, 20 gr corn starch and 10 gr magnesium stearate. Prepare sublingual THC/CBD 1:1 cannabis extract tablets containing 10 mg of THC/CBD from the above mixture.

EXAMPLE 5

Cultivation of the Plant Material

Cultivate cannabis clones of strains CN1 (who is producing about equal amounts of CBD and THC), MM9 (who is producing mostly THC), and HB3 (who is producing mostly CBD), in a soil-less growing medium consisting of 50%-50% coco coir and perlite with a fertigation system using pure food grade mineral fertilizers. The cultivation is carried out in a greenhouse with climate control according to standardized growing protocol so as to produce a consistent chemical profile over several seasons. No chemical pesticides are to be used for pest control, in order to avoid residues in the end product. Use an integrated pest management system, consisting of mechanical separation using double entries with 50 mesh insect nets, natural oils application and spreading biological pest control selected from Phytoseiulus persimilis, Diglyphus isaea, Aphidius colemani, Nesidiocoris tenuis, Cryptolaemus montrouzieri. Determine harvesting time by organoleptic testing via X100 field microscope examining trichromes colour and structure. Use further examination with a HPTLC field semi-quantitative chemical analysis kit for initial QC. Carry out harvesting by cutting the whole plant from the main stem and hanging the plant upside down on plastic wires in a temperature and humidity controlled dark room at 20 degrees Celsius and 50%-60% relative humidity using fans to create air movement in order to prevent grey mold. Strip the plants off their stems once plants have reached 10%-12% moisture content upon loss on drying, and store floral and leaf material in aluminum light proof vacuum bags as the extraction plant raw material.

Preparation Of The THC:CBD 1:1 Cannabis Extract

Using a Cannabis *sativa* clone containing about equal amounts of THC (tetrahydrocannabinol) and CBD (cannabidiol), grown under GAP conditions as determined by WHO as source of the botanical extract.

Dry, mill and then extract the plants by using super critical fluid method ($CO_2$) extraction, or non-polar extraction with butane then concentrate the botanical extract to about 70% w/w concentration of THC:CBD 1:1. This THC/CBD 1:1 70% extract is defined as the THC/CBD 1:1 cannabis extract in this application and is used in the sublingual preparations of this invention.

Preparation - Sublingual Mucoadhesive Cannabis Extract Tablets Containing 1:1 THC/CBD

Mucoadhesive Sublingual tablets preparation:

Blend the THC/CBD 1:1 cannabis extract (200 gr of the 70% w/w mixture) together with 800 gr mannitol/lactose 1:1 mixture, 30 gr PVP K-30, 200 gr Vitamin E TPGS, granulate with 600 gr ethanol USP and then dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 10 gr carbomer 934, 20 gr corn starch and 10 gr magnesium stearate. Prepare sublingual THC/CBD 1:1 cannabis extract tablets containing 10 mg of THC/CBD from the above mixture.

EXAMPLE 6

Preparation Of The THC/CBD 9:1 Cannabis Extract

Use Cannabis *sativa* clone containing about 90% of THC and 10% CBD, grown under GAP conditions as determined by WHO as source of the botanical extract.

Dry, mill and then extract the plant by using super critical fluid ($CO_2$) extraction, or non-polar extraction with butane. then concentrate the botanical extract to about 70% w/w concentration of THC &CBD (which are in ratio of about 9:1).

This THC/CBD 9:1 70% extract is defined as the THC/CBD 9:1 cannabis extract in this application and to be used in the sublingual preparations.

Preparation Of Sublingual Mucoadhesive Tablets Containing THC/CBD 9:1 Cannabis Extract

Mucoadhesive Sublingual Tablets preparation:

Blend the THC/CBD 9:1 cannabis extract (100 gr of the 70% w/w mixture) together with 800 gr mannitol/lactose 1:1 mixture,30 gr PVP K-30, 100 gr Vitamin E TPGS, 10 gr crystalline menthol, granulate with 600 gr ethanol USP and then dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 10 gr carbomer 934, 20 gr corn starch and 10 gr magnesium stearate.

Prepare sublingual THC/CBD 9:1 cannabis extract tablets containing 5 mg of THC/CBD 9:1 from the above mixture.

Example 7

Preparation Of The THC/CBD 1:9 Cannabis Extract

Using Cannabis *sativa* clone containing about 10% THC and 90% of CBD, grown under GAP conditions as determined by WHO as source of the botanical extract.

Dry, mill and then extract the plant by using supercritical fluid method ($CO_2$) extraction, or nonpolar extraction with butane. Concentrate the botanical extract to about 70% w/w concentration of THC/CBD (which are in ratio of 1:9). This THC/CBD 1:9 70% extract is defined as the THC/CBD 1:9 cannabis extract in this application and is to be used in the sublingual preparations.

Preparation of Sublingual Mucoadhesive Tablets containing THC/CBD 1:9 cannabis extract

Mucoadhesive Sublingual Tablets preparation:

Blend the THC/CBD 1:9 cannabis extract (100 gr of the 70% w/w mixture) together with 800 gr mannitol/lactose 1:1 mixture, 30 gr PVP K- 30, 100 gr Vitamin E TPGS, 10 gr crystalline menthol, then granulate with 600 gr ethanol USP and dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 10 gr PEO 301, 20 gr corn starch and 10 gr magnesium stearate.

Prepare sublingual THC/CBD 1:9 cannabis extract tablets containing 5 mg of THC/CBD 1:9 from the above mixture.

EXAMPLE 8

Preparation of Mucoadhesive Sublingual Combination of THC/CBD 1:1 cannabis Extract and Buprenorphine

Using cannabis *Sativa* clone containing about equal amounts of THC (tetrahydrocannabinol) and CBD (cannabidiol), grown under GAP conditions as determined by WHO as source of the botanical extract.

Dry, mill and then extract the plant by using super critical fluid method (CO2) extraction, or non-polar extraction with butane. Concentrate the botanical extract is to about 70% w/w concentration of CBD &THC (which are in ratio of about 1:1). This THC/CBD 1:1 70% extract is defined as the THC/CBD 1:1 cannabis extract in this application and is to be used in the sublingual preparations.

Preparation Of Sublingual Mucoadhesive Tablets Containing THC/CBD 1:1 Cannabis Extract And Buprenorphine

Mucoadhesive Sublingual Tablets Preparation:

Blend the THC/CBD 1:1 cannabis extract, (200 gr of the 70% w/w mixture) with buprenorphine HCl 50 gr, 800 gr mannitol/lactose 1:1 mixture, 30 gr PVP K-30, 200 gr Vitamin E TPGS, granulate with 600 gr ethanol USP and then dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 10 gr carbomer 934, 20 gr corn starch and 10 gr magnesium stearate.

Prepare sublingual cannabis extract tablets containing 10 mg of THC/CBD 1:1 and 2 mg buprenorphine HCl.

Example 9

Preparation Of Sublingual Mucoadhesive Film Containing THC/CBD 1:1 Cannabis Extract

Prepare cannabis API using the procedure described in Example No. 1.

Mix the THC/CBD 1:1 cannabis extract (100 gr) with 200 gr Vitamin E TPGS, 10 gr crystalline menthol, 500 gr of wet mass of hydroxypropylmethylcellulose mixed with Polyox WRS N-10, then extrude, dry and cut to a thin film containing 10 mg THC&CBD 1:1.

Example 10

Preparation Of The THC/CBD 1:1 Cannabis Extract

Using cannabis *Sativa* clone containing about equal amounts of THC (tetrahydrocannabinol) and CBD (cannabidiol), grown under GAP conditions as determined by WHO as source of the botanical extract. Dry, mill and then extract the plant by using supercritical fluid method (CO2) extraction, or non-polar extraction with butane. Concentrate the botanical extract to about 70% w/w concentration of CBD &THC (which are in ratio of 1:1). This THC/CBD 1:1 70% extract is defined as the THC/CBD 1:1 cannabis extract in this application and is to be used in the sublingual preparations.

Preparation Of Sublingual Mucoadhesive Tablets Containing THC/CBD 1:1 Cannabis Extract

Mucoadhesive Sublingual tablets preparation:

Blend the THC/CBD 1:1 cannabis extract (200 gr of the 70% w/w mixture) together with 800 gr mannitol/lactose 1:1 mixture, 30 gr PVP K-30, 200 gr Vitamin E TPGS, granulate with 600 gr ethanol USP and then dry for 45 minutes in a Glatt fluidized bed dryer, at inlet temp 50 deg C.

Mix the dry mixture with an additional amount of 200 gr mannitol, 10 gr sodium citrate & citric acid, 3 gr lemon flavor, 5 gr BHT, 5 gr BHA, 10 gr carbomer 934, 20 gr corn starch and 10 gr magnesium stearate.

Prepare sublingual THC/CBD 1:1 cannabis extract tablets containing 10 mg of THC/CBD 1:1 from the above mixture.

One exemplary embodiment of a tablet formulation of the inventive techniques:

Table 4:

| Ingredient for tablets | Mass |
| --- | --- |
| Cannabis extract 15-20 mg/tab | 15-20 mg/tab |
| Mannitol | 45-60 mg/tab |
| Lactose | 30-40 mg/tab |
| Citric acid | 0.3-0.5 $\mu$g/tab |
| Sodium citrate | 0.3-0.5 $\mu$g/tab |
| PVP | 2-3 $\mu$g/tab |
| Carbomer | 0.7-1 $\mu$g/tab |
| Starch | 1.5-2 $\mu$g/tab |
| Menthol | 0.7-1 $\mu$g/tab |
| Vitamin E TPGS | 15-20 mg/tab |
| Lemon Flavor | 0.2-0.3 $\mu$g/tab |
| Aspartame | 0.2-0.3 $\mu$g/tab |
| BHT | 0.3-0.5 $\mu$g/tab |
| Mg Stearate | 0.7-1 $\mu$g/tab |

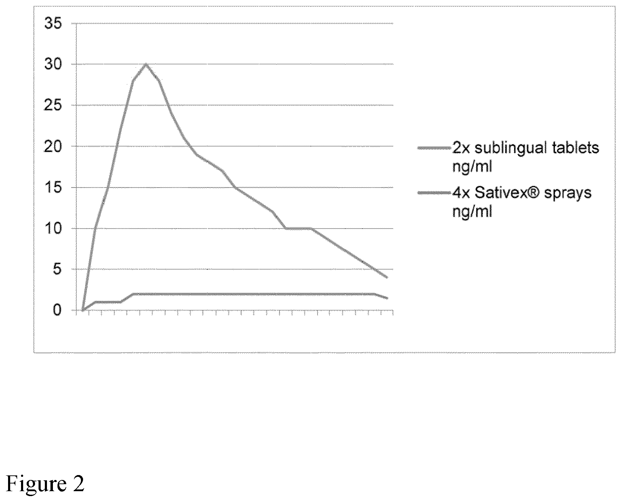

Table 5 below shows the data for the first six hours from a comparison of THC plasma levels following administration of two sublingual tablets prepared according to the exemplary embodiments, 10 mg THC, 10 mg CBD versus 4 sprays of Sativex,10.8 mg THC, 10 mg CBD. Plasma levels are presented in ng/ml. The sublingual tablets of the exemplary embodiment do not contain alcoholic residues and are non-irritable to oral and buccal mucosa. The data in Table 2 below generates the graph in **figure 2.**

Table 5

| Time (hrs) | 2x sublingual tablets ng/ml | 4x Sativex® sprays ng/ml |
|---|---|---|
| 0 | 0 | 0 |
| 0.25 | 10 | 1 |
| 0.5 | 15 | 1 |
| 0.75 | 22 | 1 |
| 1 | 28 | 2 |
| 1.25 | 30 | 2 |
| 1.5 | 28 | 2 |
| 1.75 | 24 | 2 |
| 2 | 21 | 2 |
| 2.25 | 19 | 2 |
| 2.5 | 18 | 2 |
| 2.75 | 17 | 2 |
| 3 | 15 | 2 |
| 3.25 | 14 | 2 |
| 3.5 | 13 | 2 |
| 3.75 | 12 | 2 |
| 4 | 10 | 2 |
| 4.25 | 10 | 2 |
| 4.5 | 10 | 2 |
| 4.75 | 9 | 2 |
| 5 | 8 | 2 |
| 5.25 | 7 | 2 |
| 5.5 | 6 | 2 |
| 5.75 | 5 | 2 |
| 6 | 4 | 1.5 |

Table 6 Plant cannabinoids

Cannabigerol-type (CBG): Cannabigerol (E)-CBG-C5; Cannabigerol monomethyl ether (E)-CBGM-C5 A; Cannabinerolic acid A (Z)-CBGA-C5 A; Cannabigerovarin (E)-CBGV-C3; Cannabigerolic acid A (E)-CBGA-C5 A; Cannabigerolic acid A monomethyl ether (E)-CBGAM-C5 A;

Cannabigerovarinic acid A (E)-CBGVA-C3 A

Cannabichromene-type (CBC): (±)- Cannabichromene CBC-C5; (±)-Cannabichromenic acid A CBCA-C5 A; (±)-Cannabivarichromene, (±)-Cannabichromevarin CBCV-C3; (±)-Cannabichromevarinic acid A CBCVA-C3 A

Cannabidiol-type (CBD): (-)- Cannabidiol CBD-C5; Cannabidiol momomethyl ether CBDM-C5; Cannabidiol-C4 CBD- C4; (-)- Cannabidivarin CBDV-C3; Cannabidiorcol CBD-C1;

Cannabidiolic acid CBDA-C5; Cannabidivarinic acid CBDVA-C3

Cannabinodiol-type (CBND): Cannabinodiol CBND-C5; Cannabinodivarin CBND-C3

Tetrahydrocannabinol-type (THC): Δ9- Tetrahydrocannabinol [A9-THC-C5]; Δ9-Tetrahydrocannabinol^ [A9-THC-C4]; Δ9- Tetrahydrocannabivarin [A9-THCV-C3]; A9-Tetrahydrocannabiorcol [A9-THCO-Cl]; A9-Tetrahydro-cannabinolic acid A [A9-THCA-C5 A];

A9-Tetrahydro-cannabinolic acid B [A9-THCA-C5 B]; A9-Tetrahydro- cannabinolic acid-C4 A and/or B [A9-THCA-C4 A and/or B]; A9-Tetrahydro-cannabivarinic acid A [A9-THCVA-C3 A];

(continued)

A9-Tetrahydro-cannabiorcolic acid A and/or B [A9-THCOA-Cl A and/or B (-)-A8-trans-(6aR,10aR); Δ8-Tetrahydrocannabinol [A8-THC-C5]; (-)-A8-trans-(6aR,10aR)-Tetrahydrocannabinolic acid A [A8-THCA-C5 A]; (-)- (6aS,10aR)-A9-Tetrahydrocannabinol [(-)-cis-A9-THC-C5]

Cannabinol-type (CBN): Cannabinol [CBN-C5]; Cannabinol-C4 [CBN-C4]; Cannabivarin [CBN-C3]; Cannabinol-C2 [CBN-C2]; Cannabiorcol [CBN-C1]; Cannabinolic acid A [CBNA-C5 A]; Cannabinol methyl ether [CBNM-C5]

Cannabitriol-type (CBT): (-)-(9R,10R)-trans-Cannabitriol [(-)-trans-CBT-C5]; (+)-(9S,10S)-Cannabitriol [(+)-trans-CBT-C5]; (±)-(9R,10S/9S,10R)-Cannabitriol [(±)-cis-CBT-C5]; (-)-(9R, 10R)-trans-10-0-Ethyl-cannabitriol [(-)-trans- CBT-OEt-CS]; (±)-(9R,10R/9S,10S)-Cannabitriol-C3 [(±)-trans-CBT-C3]; 8,9-Dihydroxy-A6a(10a)-tetrahydrocannabinol [8,9-Di-OH-CBT-C5];

Cannabidiolic acid A cannabitriol ester [CBDA-C5 9-OH-CBT-C5 ester]; (-)-(6aR,9S,10S, 10aR)-9,10-Dihydroxy-hexahydrocannabinol

Cannabiripsol-C5: (-)-6a,7,10a-Trihydroxy-A9-tetrahydrocannabinol; (-)-Cannabitetrol 10-Oxo-A6a (10a)-tetrahydrocannabinol [OTHC]

Cannabielsoin-type (CBE): (5aS,6S,9R,9aR)-Cannabielsoin [CBE-C5]; (5aS,6S,9R,9aR)-C3-Cannabielsoin [CBE- C3]; (5aS,6S,9R,9aR)-Cannabielsoic acid A [CBEA-C5 A];

(5aS,6S,9R,9aR)-Cannabielsoic acid B [CBEA-C5 B]; (5aS,6S,9R,9aR)-C3-Cannabielsoic acid B [CBEA-C3 B]

Cannabiglendol-C3: OH-iso-HHCV-C3

Dehydrocannabifuran [DCBF-C5]

Cannabifuran [CBF-C5]

Isocannabinoids: (-)-A7-trans-(IR,3R,6R)-Isotetrahydrocannabinol; (±)-A7-!,2-cis-(IR,3R,6S/IS,3S,6R)-Isotetrahydro-cannabivarin; (-)-A7-trans-(IR,3R,6R)-Isotetrahydrocannabivarin

Cannabicyclol-type (CBL): (±)-(laS,3aR,8bR,8cR)-Cannabicyclol [CBL-C5]; (±)-(laS,3aR,8bR, 8cR)-Cannabicyclolic acid A [CBLA-C5 A]; (±)-(laS,3aR,8bR,8cR)-

Cannabicyclovarin [CBLV-C3]

Cannabicitran-type (CBT): Cannabicitran [CBT-C5]

Cannabichromanone-type (CBCN): Cannabichromanone [CBCN-C5]; Cannabichromanone-C3 [CBCN-C3];

Cannabicoumaronone [CBCON-C5].

**Claims**

1. A composition for sublingual or buccal delivery of cannabinoid active ingredients comprising at least one cannabinoid, an emulsifying and penetration enhancing solubilizer, a permeation enhancer, and a mucoadhesive polymer;

   wherein said composition is administered sublingually or buccally, in the form of a tablet or a film,
   wherein said emulsifying and penetration enhancing solubilizer is present in an amount equal to or larger than 50% of the amount of said cannabinoid active ingredients,
   wherein said permeation enhancer comprises menthol in an amount of 1-50 mg per dose, and
   wherein said mucoadhesive polymer is present in the amount of 2-100 mg.

2. The composition of claim 1, wherein said cannabinoid active ingredients are **characterized by** at least one of the following:

   a. comprising tetrahydrocannabinol (THC) and cannabidiol (CBD) in a ratio ranging from 5:95 respectively, to 95:5 respectively;
   b. comprising THC in an amount between 0.5 and 50 mg;
   c. comprising CBD in an amount between 0.5 and 50 mg.

3. The composition of claim 1, wherein said emulsifying and penetration enhancing solubilizer comprises at least one selected from the group consisting of Vitamin E TPGS, fatty acid derivatives, terpenes, cationic surfactants, anionic surfactants, glycerides and derivatives thereof, cyclodextrin derivatives, polyols, and any combination thereof.

4. The composition of claim 1, further comprising at least one second active ingredient.

**5.** The composition of claim 4, wherein said second active ingredient comprises at least one selected from the group consisting of bupropion, buprenorphine, naloxone, methadone, at least one cannabinoid, at least one cannabinoid derivative, and any combination thereof.

**6.** The composition of claim 1, wherein said cannabinoid active ingredient has been extracted by an extraction method comprising either supercritical fluid extraction with carbon dioxide ($CO_2$) or extraction with a non-polar solvent.

**7.** The composition of claim 1, wherein said cannabinoid active ingredient is selected from the group consisting of THCA and CBDA.

**8.** The composition of claim 1, wherein said mucoadhesive polymer comprises polyethylene oxide, carbomer, polyvinylpyrrolidone, chitosan or cellulose-based polymers, in an amount ranging from 2-100 mg.

**9.** The composition of claim 1, further comprising an antioxidant selected from the group consisting of BHT, BHA, and any combination thereof.

**10.** The composition of claim 1, wherein said menthol is dissolved in oil or is in crystal form.

**11.** The composition of claim 1 for use in a method of treatment of opioid addiction or opioid dependency, said method comprising administering to a patient in need thereof of a therapeutically effective amount of said composition and optionally an additional active ingredient.

**12.** The composition of claim 11, wherein said additional active ingredient comprises at least one selected from the group consisting of benzodiazepine, buprenorphine, naloxone, methadone, bupropion, at least one addiction substance, and any combination thereof.

**13.** The composition of claim 12, wherein said at least one addiction substance comprises at least one opioid withdrawal compound.

**14.** The composition of claim 11, wherein said optionally additional active ingredient works synergistically with said at least one cannabinoid.

**15.** A composition for sublingual or buccal delivery of cannabinoid active ingredients, comprising at least one cannabinoid active ingredient in a mucoadhesive dosage form, comprising sublingual tablets or films and having greater bioavailability than an alcohol-based oral spray having 5.2mg/per spray of cannabinoid active ingredients, said composition having a plasma profile with an AUC at least 150% greater than said alcohol-based oral spray.

Figure 1

Figure 2

Plant cannabinoids

Cannabigerol-type (CBG): Cannabigerol (E)-CBG-C5; Cannabigerol monomethyl ether (E)-CBGM-C5 A;

Cannabinerolic acid A (Z)-CBGA-C5 A; Cannabigerovarin (E)-CBGV-C3; Cannabigerolic acid A (E)-CBGA-C5 A; Cannabigerolic acid A monomethyl ether (E)-CBGAM-C5 A; Cannabigerovarinic acid A (E)-CBGVA-C3 A

Cannabichromene-type (CBC): (±)- Cannabichromene CBC-C5; (±)-Cannabichromenic acid A CBCA-C5 A; (±)- Cannabivarichromene, (±)-Cannabichromevarin CBCV-C3; (±)-Cannabichromevarinic acid A CBCVA-C3 A

Cannabidiol-type (CBD): (-)- Cannabidiol CBD-C5; Cannabidiol momomethyl ether CBDM-C5; Cannabidiol-C4 CBD- C4; (-)- Cannabidivarin CBDV-C3; Cannabidiorcol CBD-C1; Cannabidiolic acid CBDA-C5; Cannabidivarinic acid CBDVA-C3

Cannabinodiol-type (CBND): Cannabinodiol CBND-C5; Cannabinodivarin CBND-C3

Tetrahydrocannabinol-type (THC): Δ9- Tetrahydrocannabinol [A9-THC-C5]; Δ9-Tetrahydrocannabinol^ [A9-THC- C4]; Δ9- Tetrahydrocannabivarin [A9-THCV-C3]; A9-Tetrahydrocannabiorcol [A9-THCO-Cl]; A9-Tetrahydro- cannabinolic acid A [A9-THCA-C5 A]; A9-Tetrahydro-cannabinolic acid B [A9-THCA-C5 B]; A9-Tetrahydro- cannabinolic acid-C4 A and/or B [A9-THCA-C4 A and/or B]; A9-Tetrahydro-cannabivarinic acid A [A9-THCVA-C3 A]; A9-Tetrahydro-cannabiorcolic acid A and/or B [A9-THCOA-Cl A and/or B (-)-A8-trans-(6aR,10aR); Δ8-Tetrahydrocannabinol [A8-THC-C5]; (-)-A8-trans-(6aR,10aR)-Tetrahydrocannabinolic acid A [A8-THCA-C5 A]; (-)- (6aS,10aR)-A9-Tetrahydrocannabinol [(-)-cis-A9-THC-C5]

Cannabinol-type (CBN): Cannabinol [CBN-C5]; Cannabinol-C4 [CBN-C4]; Cannabivarin [CBN-C3]; Cannabinol-C2 [CBN-C2]; Cannabiorcol [CBN-C1]; Cannabinolic acid A [CBNA-C5 A]; Cannabinol methyl ether [CBNM-C5]

Cannabitriol-type (CBT): (-)-(9R,10R)-trans-Cannabitriol [(-)-trans-CBT-C5]; (+)-(9S,10S)-Cannabitriol [(+)-trans- CBT-C5]; (±)-(9R,10S/9S,10R)-Cannabitriol [(±)-cis-CBT-C5]; (-)-(9R, 10R)-trans-10-0-Ethyl-cannabitriol [(-)-trans- CBT-OEt-C5]; (±)-(9R,10R/9S,10S)-Cannabitriol-C3 [(±)-trans-CBT-C3]; 8,9-Dihydroxy-A6a(10a)- tetrahydrocannabinol [8,9-Di-OH-CBT-C5]; Cannabidiolic acid A cannabitriol ester [CBDA-C5 9-OH-CBT-C5 ester]; (-)-(6aR,9S,10S, 10aR)-9,10-Dihydroxy-hexahydrocannabinol

Cannabiripsol-C5: (-)-6a,7,10a-Trihydroxy-A9-tetrahydrocannabinol; (-)-Cannabitetrol 10-Oxo-A6a(10a)-tetrahydrocannabinol [OTHC]

Cannabielsoin-type (CBE): (5aS,6S,9R,9aR)-Cannabielsoin [CBE-C5]; (5aS,6S,9R,9aR)-C3-Cannabielsoin [CBE- C3]; (5aS,6S,9R,9aR)-Cannabielsoic acid A [CBEA-C5 A]; (5aS,6S,9R,9aR)-Cannabielsoic acid B [CBEA-C5 B]; (5aS,6S,9R,9aR)-C3-Cannabielsoic acid B [CBEA-C3 B]

Cannabiglendol-C3: OH-iso-HHCV-C3

Dehydrocannabifuran [DCBF-C5]

Cannabifuran [CBF-C5]

Isocannabinoids: (-)-A7-trans-(IR,3R,6R)-Isotetrahydrocannabinol; (±)-A7-I,2-cis-(IR,3R,6S/IS,3S,6R)- Isotetrahydro-cannabivarin; (-)-A7-trans-(IR,3R,6R)-Isotetrahydrocannabivarin

Cannabicyclol-type (CBL): (±)-(laS,3aR,8bR,8cR)-Cannabicyclol [CBL-C5]; (±)-(laS,3aR,8bR,8cR)-Cannabicyclolic acid A [CBLA-C5 A]; (±)-(laS,3aR,8bR,8cR)-Cannabicyclovarin [CBLV-C3]

Cannabicitran-type (CBT): Cannabicitran [CBT-C5]

Cannabichromanone-type (CBCN): Cannabichromanone [CBCN-C5]; Cannabichromanone-C3 [CBCN-C3];

Cannabicoumaronone [CBCON-C5].

# Figure 3

| Sample Name: | THC 0.1MG/ML-1 | Injection Volume: | 10.0 |
|---|---|---|---|
| Vial Number: | RA4 | Channel: | UV_VIS_1 |
| Sequence: | 2022.09.04 Assay | Wavelength: | 220 |
| Control Program: | THC | Sample Type: | unknown |

| No. | Ret.Time min | Peak Name | Area mAU*min | Height mAU | Plates(EP) |
|---|---|---|---|---|---|
| 1 | 9.05 | THC | 141.828 | 785.012 | 15887 |

Figure 4a

| Sample Name: | 1 | Injection Volume: | 10.0 |
|---|---|---|---|
| Vial Number: | RB1 | Channel: | UV_VIS_1 |
| Sequence: | 2022.09.04 Assay | Wavelength: | 220 |
| Control Program: | SMP | Sample Type: | unknown |

| No. | Ret.Time min | Peak Name | Area mAU*min | Height mAU | Plates(EP) |
|---|---|---|---|---|---|
| 1 | 9.05 | THC | 167.270 | 910.932 | 15578 |

Figure 4b

| Sample Name: | 2 | Injection Volume: | 10.0 |
|---|---|---|---|
| Vial Number: | RB2 | Channel: | UV_VIS_1 |
| Sequence: | 2022.09.04 Assay | Wavelength: | 220 |
| Control Program: | SMP | Sample Type: | unknown |

| No. | Ret.Time min | Peak Name | Area mAU*min | Height mAU | Rel.Area % |
|---|---|---|---|---|---|
| 1 | 6.13 | n.a. | 1.246 | 9.050 | 0.51 |
| 2 | 6.42 | CBD | 4.952 | 37.580 | 2.01 |
| 3 | 6.70 | n.a. | 1.363 | 11.386 | 0.55 |
| 4 | 7.23 | CBN | 1.001 | 5.505 | 0.41 |
| 5 | 7.93 | n.a. | 8.017 | 41.895 | 3.25 |
| 6 | 9.05 | THC | 215.402 | 1159.211 | 87.38 |
| 7 | 9.60 | n.a. | 12.629 | 39.008 | 5.12 |
| 8 | 10.53 | n.a. | 1.904 | 9.058 | 0.77 |

Figure 4c

| Sample Name: | 3 | Injection Volume: | 10.0 |
|---|---|---|---|
| Vial Number: | RB3 | Channel: | UV_VIS_1 |
| Sequence: | 2022.09.04 Assay | Wavelength: | 220 |
| Control Program: | SMP | Sample Type: | unknown |

| No. | Ret.Time min | Peak Name | Area mAU*min | Height mAU | Rel.Area % |
|---|---|---|---|---|---|
| 1 | 4.68 | n.a. | 1.152 | 6.317 | 0.34 |
| 2 | 6.12 | n.a. | 1.946 | 13.126 | 0.57 |
| 3 | 6.42 | CBD | 7.068 | 53.006 | 2.08 |
| 4 | 6.70 | n.a. | 1.907 | 15.889 | 0.56 |
| 5 | 7.22 | CBN | 1.334 | 7.553 | 0.39 |
| 6 | 7.93 | n.a. | 10.539 | 57.677 | 3.11 |
| 7 | 9.05 | THC | 295.144 | 1573.485 | 87.05 |
| 8 | 9.57 | n.a. | 17.576 | 55.924 | 5.18 |
| 9 | 10.53 | n.a. | 2.387 | 11.895 | 0.70 |

Figure 4d

| Sample Name: | 4 | Injection Volume: | 10.0 |
|---|---|---|---|
| Vial Number: | RB4 | Channel: | UV_VIS_1 |
| Sequence: | 2022.09.04 Assay | Wavelength: | 220 |
| Control Program: | SMP | Sample Type: | unknown |

| No. | Ret.Time min | Peak Name | Area mAU*min | Height mAU | Rel.Area % |
|---|---|---|---|---|---|
| 1 | 6.12 | n.a. | 1.486 | 9.759 | 0.58 |
| 2 | 6.42 | CBD | 5.303 | 40.062 | 2.06 |
| 3 | 6.70 | n.a. | 1.436 | 11.851 | 0.56 |
| 4 | 7.22 | CBN | 1.001 | 5.631 | 0.39 |
| 5 | 7.93 | n.a. | 6.873 | 42.406 | 2.66 |
| 6 | 9.05 | THC | 226.094 | 1213.899 | 87.66 |
| 7 | 9.57 | n.a. | 13.932 | 40.766 | 5.40 |
| 8 | 10.53 | n.a. | 1.796 | 8.894 | 0.70 |

Figure 4e

| Sample Name: | 5 | Injection Volume: | 10.0 |
|---|---|---|---|
| Vial Number: | RB5 | Channel: | UV_VIS_1 |
| Sequence: | 2022.09.04 Assay | Wavelength: | 220 |
| Control Program: | SMP | Sample Type: | unknown |

| No. | Ret.Time min | Peak Name | Area mAU*min | Height mAU | Rel.Area % |
|---|---|---|---|---|---|
| 1 | 9.05 | THC | 166.657 | 913.080 | 98.38 |
| 2 | 9.55 | n.a. | 2.745 | 13.781 | 1.62 |

Figure 4f

| Sample Name: | 6 | Injection Volume: | 10.0 |
| Vial Number: | RB6 | Channel: | UV_VIS_1 |
| Sequence: | 2022.09.04 Assay | Wavelength: | 220 |
| Control Program: | SMP | Sample Type: | unknown |

| No. | Ret.Time min | Peak Name | Area mAU*min | Height mAU | Rel.Area % |
|-----|------|-----------|------|--------|----------|
| 1 | 9.05 | THC | 103.030 | 570.849 | 98.73 |
| 2 | 9.55 | n.a. | 1.329 | 7.011 | 1.27 |

Figure 4g

| Sample Name: | 7 | Injection Volume: | 10.0 |
|---|---|---|---|
| Vial Number: | RB7 | Channel: | UV_VIS_1 |
| Sequence: | 2022.09.04 Assay | Wavelength: | 220 |
| Control Program: | SMP | Sample Type: | unknown |

| No. | Ret.Time min | Peak Name | Area mAU*min | Height mAU | Rel.Area % |
|---|---|---|---|---|---|
| 1 | 9.05 | THC | 69.017 | 384.819 | 100.00 |

Figure 4h

| Sample Name: | 8 | Injection Volume: | 10.0 |
|---|---|---|---|
| Vial Number: | RB8 | Channel: | UV_VIS_1 |
| Sequence: | 2022.09.04 Assay | Wavelength: | 220 |
| Control Program: | SMP | Sample Type: | unknown |

| No. | Ret.Time min | Peak Name | Area mAU*min | Height mAU | Rel.Area % |
|---|---|---|---|---|---|
| 1 | 9.05 | THC | 168.995 | 906.111 | 100.00 |

Figure 4i

Figure 5a

Figure 5b

Figure 5c

Figure 5d

Figure 5e

Figure 5f

Figure 5g

Figure 5h

Figure 6a

Figure 6b

**Size Distribution Report by Number**

**Malvern**

Sample Details

Sample Name: Sample N1 3
SOP Name: mansettings.nano
General Notes: Formulation diluted

File Name: Isotopia.dts
Record Number: 130
Material RI: 1.43
Material Absorption: 0.000

Dispersant Nam... Water
Dispersant RI: 1.330
Viscosity (cP): 0.8872
Measurement Date and Time: Wednesday, September 14...

System

Temperature (°C): 25.0
Count Rate (kcps): 225.9
Cell Description: Disposable sizing cuvette

Duration Used (s): 2
Measurement Position (mm): 4.65
Attenuator: 9

Results

| | | | Size (d.nm): | % Number: | St Dev (d.n... |
|---|---|---|---|---|---|
| Z-Average (d.nm): | 933.3 | Peak 1: | 748.5 | 3.8 | 103.7 |
| PdI: | 0.821 | Peak 2: | 94.62 | 96.2 | 11.35 |
| Intercept: | 0.896 | Peak 3: | 0.000 | 0.0 | 0.000 |
| Result quality : | | | | | |

Figure 7a

## Size Distribution Report by Number

**Malvern**

Sample Details

 Sample Name: Sample N2 3
 SOP Name: mansettings_nano
 General Notes: Formulation diluted

 File Name: Isotopia.dts   Dispersant Name: Water
 Record Number: 133   Dispersant RI: 1.330
 Material RI: 1.43   Viscosity (cP): 0.8872
 Material Absorption: 0.000   Measurement Date and Time: Wednesday, September 14...

System

 Temperature (°C): 25.0   Duration Used (s): 2
 Count Rate (kcps): 146.9   Measurement Position (mm): 4.65
 Cell Description: Disposable sizing cuvette   Attenuator: 11

Results

|  |  | Size (d.nm): | % Number: | St Dev (d.n... |
|---|---|---|---|---|
| Z-Average (d.nm): 320.7 | Peak 1: | 213.1 | 95.2 | 46.11 |
| Pdl: 0.433 | Peak 2: | 1001 | 4.8 | 267.7 |
| Intercept: 0.721 | Peak 3: | 0.000 | 0.0 | 0.000 |
| Result quality : |  |  |  |  |

Figure 7b

# Size Distribution Report by Number

**Malvern**

**Sample Details**

Sample Name: Sample NO 3
SOP Name: mansettings.nano
General Notes: Formulation diluted

File Name: isotopia.dts
Record Number: 136
Material RI: 1.43
Material Absorption: 0.000

Dispersant Name: Water
Dispersant RI: 1.330
Viscosity (cP): 0.8872
Measurement Date and Time: Wednesday, September 14...

**System**

Temperature (°C): 25.0
Count Rate (kcps): 281.0
Cell Description: Disposable sizing cuvette

Duration Used (s): 2
Measurement Position (mm): 4.65
Attenuator: 0

**Results**

| | | | Size (d.nm): | % Number: | St Dev (d.n... |
|---|---|---|---|---|---|
| Z-Average (d.nm): | 248.7 | Peak 1: | 188.4 | 100.0 | 82.08 |
| PdI: | 0.319 | Peak 2: | 0.000 | 0.0 | 0.000 |
| Intercept: | 0.893 | Peak 3: | 0.000 | 0.0 | 0.000 |
| Result quality : | | | | | |

Figure 7c

# Size Distribution Report by Number
v2.2

**Malvern**

Sample Details
- Sample Name: Sample N4 3
- SOP Name: man>settings.nano
- General Notes: Formulation diluted

- File Name: Isolopia.dts
- Record Number: 130
- Material RI: 1.43
- Material Absorbtion: 0.000

- Dispersant Nam...: Water
- Dispersant RI: 1.330
- Viscosity (cP): 0.8872
- Measurement Date and Time: Wednesday, September 14...

System
- Temperature (°C): 25.0
- Count Rate (kcps): 452.1
- Cell Description: Disposable sizing cuvette
- Duration Used (s): 2
- Measurement Position (mm): 4.65
- Attenuator: 9

Results

|  | Size (d.nm): | % Number: | St Dev (d.n... |
|---|---|---|---|
| Z-Average (d.nm): 256.8 | Peak 1: 198.3 | 100.0 | 33.54 |
| Pdl: 0.308 | Peak 2: 0.000 | 0.0 | 0.000 |
| Intercept: 0.938 | Peak 3: 0.000 | 0.0 | 0.000 |
| Result quality : | | | |

Size Distribution by Number

Figure 7d

## Size Distribution Report by Number
v2.2

**Malvern**

Sample Details
    Sample Name: Sample N5 3
    SOP Name: mansettings.nano
    General Notes: Formulation diluted

    File Name: Isotopia.dts      Dispersant Nam... Water
    Record Number: 115      Dispersant RI: 1.330
    Material RI: 1.43      Viscosity (cP): 0.8872
    Material Absorption: 0.000      Measurement Date and Time: Wednesday, September 14...

System
    Temperature (°C): 25.0      Duration Used (s): 2
    Count Rate (kcps): 360.7      Measurement Position (mm): 4.65
    Cell Description: Disposable sizing cuvette      Attenuator: 8

Results

| | | Size (d.nm): | % Number: | St Dev (d.n... |
|---|---|---|---|---|
| Z-Average (d.nm): 209.9 | Peak 1: | 178.8 | 100.0 | 29.49 |
| PdI: 0.316 | Peak 2: | 0.000 | 0.0 | 0.000 |
| Intercept: 0.937 | Peak 3: | 0.000 | 0.0 | 0.000 |
| Result quality : | | | | |

Figure 7e

EP 4 378 450 A1

Size Distribution Report by Number

Malvern

Figure 7f

40

## Size Distribution Report by Number

**Malver**

Sample Details

Sample Name: Sample N7 3
SOP Name: mansettings.nano
General Notes: Formulation diluted

File Name: Isotopic.dts
Record Number: 124
Material RI: 1.43
Material Absorption: 0.000

Dispersant Nam... Water
Dispersant RI: 1.330
Viscosity (cP): 0.8872
Measurement Date and Time: Wednesday, September 1

System

Temperature (°C): 25.0
Count Rate (kcps): 339.5
Cell Description: Disposable sizing cuvette

Duration Used (s): 2
Measurement Position (mm): 4.65
Attenuator: 8

Results

| | | | Size (d.nm): | % Number: | St Dev (d.n... |
|---|---|---|---|---|---|
| Z-Average (d.nm): 234.1 | Peak 1: | | 114.2 | 65.1 | 23.74 |
| Pdi: 0.113 | Peak 2: | | 242.0 | 34.9 | 66.14 |
| Intercept: 0.044 | Peak 3: | | 0.000 | 0.0 | 0.000 |
| Result quality : | | | | | |

Figure 7g

# Size Distribution Report by Number

**Malvern**

### Sample Details

**Sample Name:** Sample N8 3
**SOP Name:** monosettings.nano
**General Notes:** Formulation diluted

| | | | |
|---|---|---|---|
| **File Name:** | Isotopia.dts | **Dispersant Name:** | Water |
| **Record Number:** | 142 | **Dispersant RI:** | 1.330 |
| **Material RI:** | 1.43 | **Viscosity (cP):** | 0.8872 |
| **Material Absorption:** | 0.000 | **Measurement Date and Time:** | Wednesday, September 14... |

### System

| | | | |
|---|---|---|---|
| **Temperature (°C):** | 25.0 | **Duration Used (s):** | 2 |
| **Count Rate (kcps):** | 162.5 | **Measurement Position (mm):** | 4.65 |
| **Cell Description:** | Disposable sizing cuvette | **Attenuator:** | 9 |

### Results

| | | | Size (d.nm): | % Number: | St Dev (d.n... |
|---|---|---|---|---|---|
| **Z-Average (d.nm):** | 962.6 | **Peak 1:** | 461.1 | 100.0 | 47.83 |
| **Pdi:** | 0.450 | **Peak 2:** | 0.000 | 0.0 | 0.000 |
| **Intercept:** | 0.993 | **Peak 3:** | 0.000 | 0.0 | 0.000 |
| **Result quality :** | | | | | |

Figure 7h

Europäisches
Patentamt

European
Patent Office

Office européen
des brevets

# EUROPEAN SEARCH REPORT

Application Number

EP 23 21 3590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/170994 A1 (SELA YORAM [IL] ET AL) 4 June 2020 (2020-06-04) * claims 1,43,46,47; examples 4-6 * | 1-15 | INV. A61K9/00 A61K9/20 A61K31/05 |
| X | US 2019/125660 A1 (THOMPSON RONALD J [US] ET AL) 2 May 2019 (2019-05-02) * claim 1; paragraphs 41,42 * | 1-15 | A61K31/485 A61K47/10 A61K47/22 |
| X | US 11 291 701 B1 (GONZALEZ DANNY S [US]) 5 April 2022 (2022-04-05) * claims 1-16 * | 1-15 | |

| | |
|---|---|
| | TECHNICAL FIELDS SEARCHED (IPC) |
| | A61K |

The present search report has been drawn up for all claims

| Place of search | Date of completion of the search | Examiner |
|---|---|---|
| The Hague | 22 March 2024 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 21 3590**

This annex lists the patent family members relating to the patent documents cited in the above-mentioned European search report.
The members are as contained in the European Patent Office EDP file on
The European Patent Office is in no way liable for these particulars which are merely given for the purpose of information.

**22-03-2024**

| Patent document cited in search report | | Publication date | Patent family member(s) | | Publication date |
|---|---|---|---|---|---|
| US 2020170994 | A1 | 04-06-2020 | CA | 3063613 A1 | 22-11-2018 |
| | | | EP | 3634452 A1 | 15-04-2020 |
| | | | US | 2020170994 A1 | 04-06-2020 |
| | | | WO | 2018211388 A1 | 22-11-2018 |
| US 2019125660 | A1 | 02-05-2019 | CA | 3151070 A1 | 10-03-2021 |
| | | | EP | 4027971 A2 | 20-07-2022 |
| | | | US | 2019125660 A1 | 02-05-2019 |
| | | | WO | 2021112812 A2 | 10-06-2021 |
| US 11291701 | B1 | 05-04-2022 | US | 11291701 B1 | 05-04-2022 |
| | | | US | 2022241360 A1 | 04-08-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 07334722
- US 16613194 B
- WO IB2018053307 A
- US 62505873 B
- US 20060257463 A

### Non-patent literature cited in the description

- **ADAMO F. et al.** Mucoadhesive Gels Designed for the Controlled Release of Chlorhexidine in the Oral Cavity. *Pharmaceutics,* 21 July 2011, vol. 3, ISSN 1999-4923, 665-679
- **WANG JUN ; SHI AIMIN ; AGYEI DOMONIC ; WANG QIANG.** Formulation of water-in-oil-in-water (W/O/W) emulsions containing trans-resveratrol. *RSC Adv.,* 2017, vol. 7, 35917-35927